Europäisches Patentamt

European Patent Office

Office européen des brevets

(11) Veröffentlichungsnummer: **0 302 011 B1**

(12) # EUROPÄISCHE PATENTSCHRIFT

(45) Veröffentlichungstag der Patentschrift: **01.02.95**

(51) Int. Cl.⁶: **C12N 5/12**, C12P 21/08, G01N 33/577, G01N 33/569, G01N 33/543

(21) Anmeldenummer: **88810487.4**

(22) Anmeldetag: **15.07.88**

(54) **Monoklonale Antikörper und Diagnoseverfahren für Phytophthora-Infektionen.**

(30) Priorität: **24.07.87 US 77616**

(43) Veröffentlichungstag der Anmeldung:
**01.02.89 Patentblatt 89/05**

(45) Bekanntmachung des Hinweises auf die Patenterteilung:
**01.02.95 Patentblatt 95/05**

(84) Benannte Vertragsstaaten:
**AT BE CH DE ES FR GB GR IT LI LU NL SE**

(56) Entgegenhaltungen:

**BIOLOGICAL ABSTRACTS, Band 82, 1986, Zusammenfassung Nr. 1126, Philadelphia, PA, US; A.R. HARDHAM et al.: "Monoclonal antibodies to isolate-, species- and genus-specific components on the surface of zoospores and cysts of the fungus"**

**BIOLOGICAL ABSTRACTS, Band 86, 1988, Zusammenfassung Nr. 45478, Philadelphia, PA, US; M.W. FERGUSON et al.: "Use of cluster analysis with monoclonal antibodies for taxonomic differentiation of phytopathogenic fungi and for screening and clustering antibodies"**

**BIOLOGICAL ABSTRACTS, Band 85, 1988, Zusammenfassung Nr. 20324, Philadelphia, PA, US; K.L. WYCOFF et al.: "Monoclonal antibodies to glycoprotein antigens of a fungal plant pathogen"**

(73) Patentinhaber: **CIBA-GEIGY AG**
**Klybeckstrasse 141**
**CH-4002 Basel (CH)**

(72) Erfinder: **Petersen, Frank**
**21 Yubas Avenue**
**Burlington, N.J. 08016 (US)**
Erfinder: **Miller, Sally**
**6313 Wyndam Road**
**Pennsauken, N.J. 08109 (US)**
Erfinder: **Rittenburg, James**
**2360 E. Rock Road**
**Perkasie, PA 18944 (US)**

**Beschreibung**

Die vorliegende Erfindung steht im Zusammenhang mit dem Gebiet der diagnostischen Pflanzenpathologie.

Im einzelnen bezieht sich die Erfindung auf monoklonale Antikörper für den immunologischen Nachweis von Phytophthora Arten, von denen bekannt ist, dass sie auslösender Faktor für eine Reihe von Pflanzenkrankheiten sind.

Ganz allgemein sind Pilze häufig die Ursache für zahlreiche Pflanzenkrankheiten.

Als Diskussionsgrundlage kann man die Pilze generell in drei grosse taxonomische Klassen einteilen: Ascomyceten, Basidiomyceten und Phycomyceten.

Ascomyceten

Die Mitglieder dieser Gruppe sind durch das Vorhandensein eines Ascus charakterisiert, einer spezialisierten reproduktiven Struktur, in der sowohl die Meiose als auch die sexuelle Sporenbildung stattfindet.

Als Beispiele allgemein bekannter Pflanzenkrankheiten, für die Ascomyceten als Verursacher bereits identifiziert sind, seien genannt: echte Mehltaue an Getreide, Früchten und anderen Kulturpflanzen; das Ulmensterben ("Dutch elm disease"); das Mutterkorn des Getreides; die Monilia - Fruchtfäule bei Steinobst; die Blattfleckenkrankheit bei Rosen sowie der Apfelschorf.

Basidiomyceten

Die Mitglieder dieser Gruppe können anhand ihrer für die Bildung sexueller Sporen verantwortlichen Struktur, dem sogenannten Basidium, identifiziert werden.

Pathogene Formen schliessen Brandpilze, Rostpilze und fleischige Spezies, wie etwa die Hutpilze, ein.

Als Beispiele seien genannt: der Weizenrost, die Blasenkrankheit der Kiefer, der Apfelrost, sowie die Brandpilze, welche Erkrankungen bei Mais, Hafer, Gerste, Zwiebeln und Weizen verursachen.

Phycomyceten

Die Phycomyceten umfassen Formen, die als primitiver angesehen werden als die Mitglieder der Ascomyceten oder der Basidiomyceten.

Das wesentliche morphologische Unterscheidungsmerkmal ist das Fehlen von Querwänden im Phycomycetenmycel.

Beispiele für Pflanzenkrankheiten, die durch Mitglieder aus dieser Klasse verursacht werden umfassen unechte Mehltaue beim Wein und anderen Wirtspflanzen sowie Wurzelfäule und Braunfäule bei Kartoffel und Tomate.

Im Zusammenhang mit der vorliegenden Erfindung sind in erster Linie Mitglieder der Phycomycetengattung Phytophthora ("Pflanzenzerstörer") von Interesse.

Diese Gattung erhielt ihren Namen 1876 durch Anton de Bary, in der Absicht das verursachende Prinzip (Phytophthera infestans) der Kartoffelfäule zu beschreiben, einer Krankheit, die in Irland in der Mitte des 19. Jahrhunderts zu ausgedehnten Hungersnöten führte. Bis heute sind 43 weitere Phytophthora-Arten beschrieben worden, die allesamt pathogen für Pflanzen sind. Eine Reihe dieser Arten wird noch weiter unterteilt in Varietäten, Spezialformen und/oder Rassen.

Es existieren eine Reihe ausgezeichneter Monographien und Bücher, die über die Taxonomie, Biologie, Oekologie und Pathologie von Arten, die in dieser Gattung zusammengefasst sind, Auskunft geben (siehe z.B. Waterhouse, G.M., Mycological Papers No. 122 (1970) Erwin, D.C., Bartnicki-Garcia, S. and Tsao, P.H. (Eds.) Phytophthora: Its Biology, Taxonomy, Ecology and Pathology (1983) Am. Phytopathological Soc., St. Paul, MN).

Die Gattung Phytophthora gehört zur Familie Pythiaceae, deren anderes Mitglied die Gattung Pythium ist.

Obwohl die Gattung Pythium von der Anzahl der beschriebenen Arten her betrachtet die grössere Gattung ist, besitzt die Gattung Phytophthora aber einen höheren Prozentsatz an Arten, die Erkrankungen verursachen, welche unter ökonomischen Gesichtspunkten betrachtet von grosser Bedeutung sind. Einige der bedeutsameren Arten sind in Tabelle 1 zusammengefasst:

Tabelle 1

| Verschiedene Pflanzenkrankheiten für die Phytophthora Arten als ethiologisches Agenz erkannt sind. | |
|---|---|
| Arten | Krankheit |
| P. cactorum | Wurzel- und Kragenfäule beim Apfel |
| P. capsici | Wurzelfäule beim Pfeffer |
| P. cinnamomi | Jarrah-Wurzelfäule, Avocado-Wurzelfäule sowie Erkrankungen verschiedener hölzener Zierpflanzenarten |
| P. citrophthora | Wurzelfäule bei Zitrusfrüchten |
| P. colocasiae | Blatt- und Rhizomfäule von Taro |
| P. fragariae | Erdbeerfäule |
| P. infestans | Kartoffelfäule, Braunfäule bei Tomaten |
| P. megasperma | Wurzel- und Kragenfäule bei Apfel, Kirsche und anderen Obstarten |
| P. megasperma f.sp. glycinea | Wurzelfäule |
| P. megasperma f.sp. medicaginis | Wurzelfäule |
| P. palmivora | Braunfäule bei Kakao |
| P. parasitica | Wurzelfäule bei Zitrusfrüchten |
| P. parasitica nicotianae | Tabakwurzelfäule |
| P. phaseoli | Wurzelfäule bei Bohnen |
| P. syringae | Apfelfäule |

Das Diagnostizieren von Erkrankungen die von Phytophthora spp. verursacht werden, wird oft durch das Fehlen leicht erkennbarer und/oder eindeutiger Symptome erschwert. Ausserdem ist es oft nicht möglich das Pathogen von dem infizierten Gewebe zu isolieren und auf Nährmedien zu kultivieren.

Dies ist von besonderer Bedeutung bei Phytophthora-Erkrankungen, welche die Pflanzenwurzeln befallen. Hier stören in erster Linie nichtpathogene oder aber nur schwach pathogene Phythium spp., die sich in oder auf den Wurzeln befinden. Sie machen die Isolierung von Phytophthora spp. unmöglich, da diese auf besagten Nährmedien in der Regel sehr viel langsamer wachsen als Pythium-Arten. Es wurden daher Köder-Techniken für die Isolierung von Phytophthora spp. aus Bodenproben oder von Pflanzenwurzeln entwickelt, die aber in der Regel zeitaufwendig und ebenfalls anfällig für Kontaminationen sind.

Im Falle der Phytophthora Wurzel- und Stengelfäule von Sojabohnen, die durch P. megasperma f.sp. glycinea verursacht wird, kann das Pathogen nur durch Anlocken von den Wurzeln isoliert werden. Daraus folgt, dass Wurzelerkrankungen beim Fehlen offensichtlicher Symptome am Stengel oft unerkannt bleiben oder aber einer Fehldiagnose unterliegen.

Ein monoklonaler Antikörper, der in der Lage ist Phytophthora spp. von anderen Mikroorganismen, insbesondere aber von Phythium spp. in Pflanzengewebe mit Hilfe eines einfach anzuwendenden Immunoassays zu unterscheiden, würde das Erkennen ertragsvermindernder "verborgener Phytophthora Arten" in Pflanzen ermöglichen.

Diese Aufgabe konnte jetzt im Rahmen der vorliegenden Erfindung überraschenderweise gelöst werden, durch die Entwicklung eines einfach anzuwendenden Immunassays für die schnelle Diagnose von Phytophthora--Erkrankungen in infiziertem pflanzlichem Gewebe unter Verwendung der an sich bekannten Hybridoma/monoklonalen Antikörper-Technologie.

Die Verwendung hybrider somatischer Zellinien als Quelle für Antikörper gegen ganz bestimmte Antigene geht auf die Arbeiten von Köhler und Milsten (Nature 256: 495-97, 1975) zurück.

Die Antikörper, die sich mit dem dort beschriebenen Verfahren gewinnen lassen, unterscheiden sich sehr stark von denjenigen, die man aus Antiseren konventionell immunisierter Tiere erhält.

Jede dieser Hybrid-Zellinien synthetisiert ein homogenes Immunglobulin, das nur einen einzigen Vertreter aus der grossen Anzahl möglicher Antikörper repräsentiert, die von einem Tier als Antwort auf ein Antigen in vivo synthetisiert werden kann.

Da jeder Immunglobulin-produzierende Klon durch einen einzigen Typ von Antikörpern charakterisiert wird, hat sich die Bezeichnung monoklonale Antikörper eingebürgert.

Die Vorteile monoklonaler Antikörper sind zahlreich; sie können in grosser Anzahl erhalten werden, die Herstellung ist homogen im Hinblick auf die Antigen-Reaktivität und ändert sich auch nicht im Verlaufe der Zeit. Das Prinzip der Hybridoma/Monoklonalen Antikörper-Technologie gründet sich auf die Beobachtung, dass beim Verschmelzen zweier somatischer Zellen die resultierende Hybridzelle charakteristische Merkmale beider Elterntypen aufweist.

Im Falle der monoklonalen Antikörperproduktion stammt die Fähigkeit zur Synthese des spezifischen Antikörpers von einer immunkompetenten Zelle (gewöhnlich einer Milzzelle), die einem zuvor immunisierten Donor-Tier entnommen wurde, während die Fähigkeit zur kontinuierlichen Zellteilung in Kultur durch den anderen Fusionspartner, eine Tumorzellinie (oft ein Myeloma) beigesteuert wird.

Anfänglich wurden die Fusionen dadurch kompliziert, dass auch die Myelomazellinien monoklonale Antikörper synthetisierten, so dass das Hybrid zwei Typen monoklonaler Antikörper produzierte, einen, der seinen Ursprung in der Myeloma-Zelle hatte und einen zweiten, der durch die genetische Information der immunokompetenten Zelle bestimmt wurde.

In der Folge wurden daher Tumorzellen verwendet, die selbst keine monoklonale Antikörper herstellen können, e.g. SP2/0-Ag14 oder X63-Ag8.653, wodurch die Analyse der resultierenden Fusionsprodukte sehr stark vereinfacht wurde.

Ein weiterer technischer Aspekt des Verfahrens betrifft die Erarbeitung einer gangbaren Methode für das Auslesen erfolgreicher Fusionsereignisse (Hybridzellen) aus den 2 Typen von Elternzellen. Routinemässig werden eine Million und mehr Zellen von jedem Elterntyp in dem Fusionsprotokoll verwendet. Da die Fusion nicht mit einer 100 %igen Frequenz erfolgt, kann es zu einem schwierigen Unterfangen werden zu versuchen, die Fusionsprodukte von dem hohen Hintergrund an nicht fusionierten oder mit sich selbst fusionierten Elternzellen abzutrennen.

Wie bereits zuvor erwähnt entstehen die Hybridomazellen durch Fusion kurzlebiger Antikörper produzierender (Milz) Zellen sowie langlebiger Myeloma Zellen.

Das gewünschte Resultat ist eine langlebige Zellinie, die Antikörper produziert. Da die Milzzellen nur eine begrenzte Lebenszeit in Kultur besitzen, kann man daher im Prinzip einfach abwarten, bis alle nichtfusionierten sowie alle mit sich selbst fusionierten Milzzellen abgestorben sind. Dann bleibt jedoch immer noch die Aufgabe bestehen, die langlebigen Antikörper-produzierenden Zellen von den langlebigen kein Antikörper produzierenden Zellen abzutrennen.

Ein gängiges System für die Selektion von Hybridzellen ist das sogenannte HAT-Selektionssystem.

Dieses System beinhaltet die Verwendung des Enzyms Hypoxanthinguaninphosphoribosyltransferase (HGPRT). Dieses Enzym ist Bestandteil des Purin "Salvage Pathway" in Säugerzellen.

Diese Zellen sind darüberhinaus auch in der Lage Purine de novo zu synthetisieren.

Unter normalen Umständen arbeiten wahrscheinlich beide Synthese Wege bis zu einem gewissen Grade parallel.

Falls eine Zelle jedoch kein HGPRT besitzt, ist der "Salvage Pathway" blockiert und die Purine müssen aus nicht-Purin Material hergestellt werden.

Die chemische Verbindung 8-Azaguanin ist ein sogenannter Antimetabolit, der dem Purin Guanin strukturell sehr ähnlich ist und dieses daher in einigen seiner normalen Reaktionen ersetzen kann.

Azaguanin wird in die DNA eingebaut, was zu einer Beeinträchtigung des normalen Wachstumsverhaltens und schliesslich zum Zelltod führt. Da Azaguanin über den "Salvage Pathway" ersetzt werden muss, können alle diejenigen Zellen, die keine HGPRT Aktivität besitzen, Azaguanin nicht verwerten und daher in dessen Gegenwart wachsen.

Ein selektives System, das mit demselben Enzym aber unter umgekehrten Vorzeichen arbeitet, indem in diesem Fall HGPRT positive Zellen selektiert werden, ist bei J.W. Littlefield (Science, 145: 709 (1964) beschrieben.

Es wird als HAT-System bezeichnet und beinhaltet Hypoxanthin, Aminopterin und Thymidin (HAT-Medium). Aminopterin ist ein Antimetabolit, der die de novo Purin-Synthese sowie die Methylierung von Desoxyuridylat zu Thymidylat hemmt.

Hypoxanthin kann als Hilfspurin fungieren für den Fall, dass Aminopterin die de novo Purin-Synthese blockiert, während Thymidin die Notwendigkeit einer Methylierung von Desoxyuridylat überflüssig macht.

Daher werden in Gegenwart von Aminopterin alle HGPRT positiven Zellen proliferieren, während die HGPRT negativen Zellen absterben.

In dem Hybridsystem, das im Rahmen dieser Erfindung für die Selektion verwendet wird, sind die Myeloma-Zellen vorzugsweise resistent gegenüber Azoguanin und empfindlich gegenüber Aminopterin, d.h. sie sind HGPRT negativ. Die Antikörper-produzierenden Zellen dagegen sind HGPRT positiv.

Durch Fusion der Zellen und Kultivierung in einem HAT-Medium ohne Azoguanin (HT-Medium), können die erfolgreich miteinander fusionierten Zellen selektiert werden, da die Myeloma Zellen, die für die Proliferation verantwortlich sind, nur in Gegenwart einer HGPRT-Aktivität wachsen können und diese Aktivität von der HGPRT positiven Zellinie geliefert werden muss.

Die HGPRT positiven Antikörper-produzierenden Zellinien werden in diesem Medium nicht abgetötet. Sie überleben eine bestimmte Zeit, proliferieren aber nicht.

Damit wird durch Fusion der Zellen in einem HAT-Medium ein System geschaffen, in welchem zwar die Myeloma Zellen und die Antikörper produzierenden Zellen für einen Zeitraum wachsen können, der ausreicht für die Produktion von Hybrid Zellen, in dem aber nur die Hybrid-Zellen überleben und proliferieren können.

Nach der Selektion wird jeder Hybridomaklon auf seine Fähigkeit zur Bildung des jeweils interessierenden spezifischen Antikörpers hin untersucht.

Die Hybridoma/Monoklonale Antikörper Technologie war ausserordentlich erfolgreich. Ein Hinweis in dieser Richtung liefert die Tatsache, dass innerhalb des Klassifikationssystems des U.S. Patentamts eine eigene Sub-Klasse für monoklonale Antikörper (935/100) eingeführt wurde.

Zur Illustrierung der Aktivitäten auf dem Gebiet der monoklonalen Antikörper Technologie seien die folgenden US-Patente genannt: US-Patent No. 4,196,265, das auf die Produktion von monoklonalen Antikörpern gegen Viren gerichtet ist; US-Patent No. 4,404,279, das ein Verfahren zur Kultivierung von Hybridomas sowie zur Erhöhung der Hybridisierungsrate beschreibt; und US Patent 4,427,653, das ein Verfahren zur Herstellung monoklonaler Antikörper beschreibt, worin die Antigen-Präparation vor der Immunisierung durch bestimmte monoklonale Antikörper absorbiert wird.

Im folgenden sind eine Reihe von Antigenen aufgeführt, gegen die bereits monoklonale Antikörper hergestellt wurden, ohne dass diese Auflistung einen Anspruch auf Vollständigkeit erhebt:

Treponema pallidum (EPO-83302898.8), Hepatitis Antigen (EPO-83103858.3), anti-H.-Y. (EPO-83301214.9), Linsenepithelzellen (83301176.0), Karzinoembryonalantigen (PTC W081101469), Urokinase (EPO-83100190.4), Herpes (EPO-83400074.7), Ratten Hepatocyten (82306409.2), Schistosoma mansoni (PCT W083/01837), Leishmania (PCT-W083/01785), Transferrin Rezeptor Glycoprotein (EPO-82305658.5), Rheumatoid-Faktor (PCT W083/01118), Zelloberflächenantigen eines humanen Nierenkarzinoms (EPO-83107355.8), Alpha Interferon (PCT W081/02899), T-Zell Antigen (EPO-81300047.8), humane Suppressor T-Zellen (EP-O 80304348.8).

Im Zusammenhang mit Pflanzenkrankheiten wurden von Hsu H.T., et al. (ASM News, 50(3): 91-101, 1984) insgesamt 18 Spezies von Pflanzenviren aufgelistet, gegen die monoklonale Antikörper entwickelt wurden, darunter "Carnation Etched Ring Virus", "Potato Leaf Roll Virus", "Southern Bean Mosaik Virus", Tabak Mosaik Virus, "Tomato Ring Spot Virus" sowie "Tulip Breaking Virus".

Monoklonale Antikörper gegen Pilze wurden bisher in erster Linie als Hilfsmittel für die Diagnose humaner Erkrankungen entwickelt.

So beziehen sich beispielsweise die beiden UK Patentanmeldungen Nr. GB 2138444A und GB 2138445A auf monoklonale Antikörper, die mit Candida und Aspergillus Arten reagieren.

Gegenstand der vorliegenden Erfindung ist ein monoklonaler Antikörper, der spezifisch mit Vertretern der Pilzgattung Phytophthora reagiert sowie Verfahren zu dessen Herstellung.

Der erfindungsgemässe Antikörper kann vorzugsweise für einen umfassenden Nachweis von Phytophthora-Infektionen verwendet werden.

Polyklonale Antiseren wurden bereits gegen verschiedene Phytophthora-Spezies hergestellt, zum einen für die Beantwortung taxonomischer Fragestellungen (D.M. Halsall, J. Gen. Microbiol., 94: 149-158, 1976) zum anderen für die Untersuchung zahlreicher Aspekte der Wirts-Parasit Wechselwirkungen [P. Moesta, H. Griesbach und E. Zeigler (1983), Ein. J. Cell Biol., 31: 167-169, 1983] sowie der Pathogen Oekologie (J.D. MacDonald und J.M. Duniway, Phytopathology, 69: 436-441 1979).

Monoklonale Antikörper wurden weiterhin hergestellt durch Ayers, et al. [In: Arnetzen, G. and Ryan, C. (Ed.), Molecular Strategies for Crop Protection-UCLA Symposium on Molecular and Cellular Biology, New Series, Vol. 48 (1986) New York: Alan Liss, Inc., p. 71-81; ebenso Goodell, et al. (1985) In: Key, J.L., Kosuge, T. (Ed.); Cellular and Molecular Biology of Plant Stress, UCLA Symposis on Molecular and Cellular Biology, New Series, Vol. 22, New York: Alan Liss, Inc., p. 447-457] gegen extrazelluläres Glycoprotein oder gereinigte Zellwände von P. megasperma f.sp. glycinea Mycel in Zusammenhang mit einem erfolglosen Versuch die Rassenspezifischen Komponenten zu definieren, die mit der Induktion der Resistenzantwort in Sojabohnen-Pflanzen in Zusammenhang stehen.

Hardham, et al. (Exp. Mycol. 9: 264-268, 1985) produzierte monoklonale Antikörper gegen Glutaraldehyd/Paraformaldehyd fixierte Zoosporen oder enzystierte Zoosporen von P. cinnamomi. Es wurden demnach bereits eine Reihe verschiedener monoklonaler Antikörper mit unterschiedlicher Spezifität hergestellt. Keiner dieser Antikörper wurde jedoch im Zusammenhang mit dem Nachweis von Erkrankungen bei Pflanzen verwendet.

Obwohl also andere monoklonale Antikörper existieren, gab es bis zu diesem Zeitpunkt keinen diagnostischen Test für den Nachweis von Phytophthora Infektionen in erkrankten Pflanzen. Dies kann zumindest teilweise der Tatsache zugeschrieben werden, dass nicht alle monoklonalen Antikörper für die Verwendung in dem gebräuchlichsten Assay-Typ, i.e. einem doppelten Antikörper-Assay, geeignet sind.

Es gibt verschiedene Gründe, warum ein monoklonaler Antikörper für diesen speziellen Zweck nicht geeignet sein kann.

Um beispielsweise das interessierende Antigen präzise nachweisen zu können, muss der zweite Antikörper entweder an ein anderes Epitop des Antigens binden als der erste Antikörper oder aber der Antikörper muss gegen ein multiples Epitop gerichtet sein.

Weiterhin muss der Antikörper in der Lage sein, Antigene nachzuweisen, die mit Phytophthora infiziertem Pflanzengewebe assoziiert sind.

Es ist in diesem Zusammenhang nicht ungewöhnlich, dass man Antikörper produziert, die gegen Determinanten gerichtet sind, welche zwar in kultivierten Organismen vorhanden sind, nicht aber in infiziertem Gewebe oder aber dort zumindest nicht diagnostizierbar sind.

Schliesslich ist es möglich, dass bestimmte Antikörper zwar unter Langzeitbedingungen (e.g. 24 Stunden) im Labor in der Lage sind Phytophthora nachzuweisen, ohne jedoch diese Aufgabe auch in einem Immunassay zu erfüllen, das aus kommerzieller Sicht betrachtet brauchbar ist, also ein Immunassay, das innerhalb einer Zeitspanne von etwa 20 Minuten ein genaues positives oder negatives Resultat liefert. Unter diesem Gesichtspunkt betrachtet weist keiner der zuvor beschriebenen monoklonalen Antikörper, die auf ihre Eignung in einem diagnostischen Test-Kit hin untersucht wurden, die charakteristischen Eigenschaften auf, die notwendig sind für eine Verwendung in einem schnellen und ökonomischen doppelten Antikörper-Immunassay.

All die zuvor erwähnten Probleme und Schwierigkeiten konnten jetzt im Rahmen dieser Erfindung durch die Anwendung einfacher Verfahrensmassnahmen gelöst werden, i.e. durch die Verwendung monoklonaler Antikörper, die von einem erfindungsgemässen Hybridoma produziert werden, zum immunologischen Nachweis von Phytophthora Infektionen.

Im einzelnen bezieht sich die vorliegende Erfindung auf ein Hybridoma das einen monoklonalen Antikörper produziert, der mit mindestens einem pathogenen Stamm der Gattung Phytophthora reagiert, der aber im wesentlichen keine Reaktion mit Stämmen aus der Gattung Pythium zeigt und der in der Lage ist Phytophthora in erkranktem Gewebe in einem zwei Antikörper umfassenden Immunassay nachzuweisen, sowie ein Verfahren zur Herstellung besagter Hybridomas.

Im einzelnen handelt es sich dabei um ein Verfahren zur Herstellung eines Hybridoma, das dadurch gekennzeichnet ist, dass man

a) eine immunkompetente Zelle aus einem zuvor immunisierten Donor-Tier isoliert

b) mit einer zur kontinuierlichen Zellteilung befähigten Tumorzellinie fusioniert und

c) das entstehende Fusionsprodukt isoliert.

Ebenso verwendbar in dem erfindungsgemässen Verfahren zum immunologischen Nachweis von Phytophthora Infektionen sind Mutanten und Varianten besagter Hybridomas, die noch einen monoklonalen Antikörper produzieren, der mit mindestens einem Stamm der Gattung Phytophthora reagiert, der aber im wesentlichen keine Reaktion mit Stämmen aus der Gattung Pythium zeigt und die sich mit Hilfe an sich bekannter Verfahren sehr einfach aus dem vorliegenden Ausgangsmaterial herstellen lassen.

Unter dem Begriff "reagieren mit" bzw. "Reaktion", soll in diesem konkreten Zusammenhang, sowie im Verlaufe der gesamten Beschreibung und in den Ansprüchen die Fähigkeit des Antikörpers verstanden werden (oder das Fehlen dieser Fähigkeit) mit einem bestimmten Antigen einen binären Komplex auszubilden, im vorliegenden Fall mit Antigenen der Gattung Phytophthora.

Die vorliegende Erfindung umfasst ebenso die auf diese Weise produzierten Antikörper selbst sowie ein Verfahren und einen Kit für den Nachweis einer Phytophthora Infektion unter Verwendung des erfindungsgemässen monoklonalen Antikörpers als Reagenz.

Insbesondere betrifft die vorliegende Erfindung einen monoklonalen Antikörper, der mit mindestens einem pathogenen Stamm aus der Gattung Phytophthora reagiert, der aber im wesentlichen keine Reaktion mit Stämmen aus der Gattung Pythium zeigt und der in der Lage ist, Phytophthora in erkranktem Gewebe in einem zwei Antikörper umfassenden Immunassay nachzuweisen.

Ebenso umfasst von der vorliegenden Erfindung ist ein Verfahren zur Herstellung eines solchen monoklonalen Antikörpers, das dadurch gekennzeichnet ist, dass man ein Hybridoma, welches einen monoklonalen Antikörper produziert, der mit mindestens einem pathogenen Stamm aus der Gattung Phytophthora reagiert, der aber im wesentlichen keine Reaktion mit Stämmen aus der Gattung Pythium zeigt und der in der Lage ist, Phytophthora in erkranktem Gewebe in einem zwei Antikörper umfassenden Immunassay nachzuweisen, in vitro oder in vivo massenkultiviert.

Ein weiterer Gegenstand dieser Erfindung betrifft ein Verfahren zum Nachweis von Phytophthora Antigen in einer Probe, die dieses besagte Antigen enthält, das gekennzeichnet ist:

a) durch die Ausbildung eines binären Komplexes zwischen dem Antigen und einem monoklonalen oder polyklonalen Antikörper, der in der Lage ist mit diesem besagten Antigen zu reagieren;

b) durch Ausbildung eines Dreifach-Komplexes, indem man besagten binären Komplex mit einem weiteren monoklonalen oder polyklonalen Antikörper in Kontakt bringt;

c) durch den Nachweis eines solchen Dreifachkomplexes, aufgrund eines wahrnehmbaren Signals, das durch ein analytisch nachweisbaren Reagenz produziert wird, das gegebenenfalls an einen dritten Antikörper konjugiert vorliegen kann;

wobei zumindest einer dieser besagten Antikörper ein erfindungsgemässer Antikörper ist.

Das analytisch nachweisbare Reagenz ist vorzugsweise mit einem zweiten Antikörper konjugiert; es kann aber auch gegebenenfalls an einen dritten, anti-Immunglobulin-Antikörper konjugiert sein.

Ein letzter Gegenstand dieser Erfindung betrifft die Bereitstellung eines Test-Kits für die immunologische Diagnose von Phytophthora-Infektionen bei Pflanzen, der gekennzeichnet ist durch einen Carrier, welcher kompartimentiert ist, so dass darin in enger räumlicher Beziehung zueinander angeordnet sind:

a) ein fester Träger, der einen ersten monoklonalen oder polyklonalen Antikörper gebunden enthält, welcher in der Lage ist einen binären Komplex mit Phytophthora Antigen auszubilden; und

b) ein System zum Nachweis eines binären Komplexes, das sich zusammensetzt aus einem zweiten Antikörper, der in der Lage ist einen Dreifach-(tertiären) Komplex auszubilden, indem er mit dem binären Komplex reagiert;

wobei zumindest einer der besagten Antikörper ein erfindungsgemässer Antikörper ist.

Die vorliegende Erfindung betrifft Verfahren zur Herstellung von monoklonalen Antikörpern gegen Phytophthora megasperma f.sp. glycinea, die monoklonalen Antikörper per se, Hybridoma-Zellinien, die in der Lage sind besagte Antikörper zu produzieren, Verfahren zur Herstellung besagter Hybridoma-Zellinien sowie Verfahren und Kits, in welchen die monomolekularen Antikörper für die Diagnose von Phytophthora Infektionen in pflanzlichem Gewebe verwendet werden.

Auch wenn, wie aus der vorangegangenen Diskussion klar geworden ist, monoklonale Antikörper für Phytophthora bereits bekannt waren, so existierte aber bisher kein Verfahren, das die Verwendung solcher Antikörper in einem diagnostischen Test-Kit zum Nachweis von Phytophthora in situ, d.h. im erkrankten pflanzlichen Gewebe ermöglicht hätte.

Die Schwierigkeiten beruhen zum Teil auf der Tatsache, dass nicht alle verfügbaren Antikörper die notwendige Spezifität zur Differenzierung von Phytophthora gegenüber Pythium aufweisen. Darüberhinaus sind viele Antikörper, selbst wenn sie die erforderliche Spezifität besitzen, für die Verwendung in einem Test-Kit dennoch nicht geeignet.

Bevorzugt im Rahmen dieser Erfindung ist ein Testverfahren, bei dem ein doppeltes Antikörper System direkt auf das vermutlich erkrankte pflanzliche Gewebe aufgebracht wird.

Der Phytophthora-spezifische Antikörper, der vorzugsweise in Verbindung mit einem festen Träger vorliegt, wird auf das pflanzliche Gewebe aufgebracht; anschliessend wird ein zweiter markierter Antikörper hinzugefügt, um das Vorhandensein eines Phytophthora Antigen-Antikörper Komplexes nachweisen zu können. Ueberraschenderweise hat es sich gezeigt, dass keiner der getesteten und bereits vorbeschriebenen Phytophthora Antikörper für einen exakten Nachweis von Phytophthora in dieser Art von Assay geeignet war.

Der erfindungsgemässe Antikörper dagegen ist im Gegensatz zu anderen, bekannten Antikörpern in der Lage, bei Verwendung eines doppelten Antikörper-Systems Phytophthora in erkranktem Gewebe nachzuweisen.

Die monoklonalen Antikörper dieser Erfindung gehören zu einer vergleichsweise seltenen Subklasse von Immoglobulinen, nämlich der IgG2b Subklasse. Antikörper aus dieser Subklasse lassen sich verhältnismässig einfach isolieren und reinigen, da sie bei geringer Ionenstärke aus einer Lösung ausfallen.

Unter einem geeigneten Antikörper ist im Rahmen dieser Erfindung ein Antikörper zu verstehen, der ein breites Reaktivitätsspektrum innerhalb der Gattung Phytophthora besitzt und in der Lage ist zumindest mit einem pathogenen Stamm, vorzugsweise aber mit mindestens 5 Stämmen und ganz besonders bevorzugt mit mindestens 15 Stämmen der Gattung Phytophthora zu reagieren, ohne dabei aber in nennenswertem Umfang Reaktionen mit Stämmen der Gattung Pythium einzugehen (siehe Tabelle 3).

Ein bevorzugter monoklonaler Antikörper, der alle die oben erwähnten Anforderungen erfüllt, wird von einem Hybridoma produziert, das bei der American Type Culture Collection in Rockville, Maryland, unter der Eingangsnummer HB 9353 (PH 4830) hinterlegt ist. Bei PH 4830 handelt es sich um den bevorzugten Antikörper, was die Verwendung in einem diagnostischen Test-Kit zur Ueberprüfung auf das Vorliegen einer Phytophthora Infektion in situ angeht.

Die vorliegende Erfindung umfasst auch die Verwendung eines monoklonalen Antikörpers, der mit mindestens einem pathogenen Stamm aus der Gattung Phytophthora reagiert, der aber im wesentlichen keine Reaktion mit Stämmen aus der Gattung Pythium zeigt und der in der Lage ist, Phytophthora in erkranktem Gewebe in einem zwei Antikörper umfassenden Immunoassay nachzuweisen, in einem System zum Nachweis von Phytophthora Infektionen in erkranktem Gewebe. Dementsprechend wird eine Probe pflanzlichen Materials, das im Verdacht steht den Organismus zu enthalten, mit einem ersten Antikörper, der spezifisch mit einer antigenen Determinanten des nachzuweisenden Organismus reagiert, in Kontakt gebracht. Vorzugsweise handelt es sich bei besagtem Antikörper um einen an einem festen Träger, wie z.B. an den Wänden einer Mikrotiterplatte immobilisierten Antikörper.

Der Antikörper kann ein monoklonaler Antikörper oder aber ein Bestandteil eines polyklonalen Serums sein, das mit Phytophthora reagiert.

Nach Entfernen des nicht umgesetzten Materials durch Auswaschen wird der verbleibende binäre Komplex (Antigen-Antikörper-Komplex) mit einem monoklonalen oder polyklonalen Antikörper in Kontakt gebracht, der spezifisch mit dem nachzuweisenden Antigen reagiert.

Durch den Kontakt des immobilisierten binären Komplexes mit dem zweiten monoklonalen Antikörper entsteht ein tertiärer Komplex (Antikörper-Antigen-Antikörper). Zumindest bei einem der Antikörper, die am Aufbau des tertiären Komplexes beteiligt sind, handelt es sich um einen erfindungsgemässen Antikörper. Nach dem Entfernen all derjenigen sekundären Antikörper, die nicht an den sekundären Komplex gebunden haben, durch Auswaschen, kann der entstandene tertiäre Komplex mit Hilfe einer Reihe von verschiedenen analytischen Techniken nachgewiesen werden. Der zweite Antikörper kann z.B. direkt mit einem analytisch bestimmbaren Reagenz markiert werden, so dass der gebildete tertiäre Komplex dann anhand eines durch dieses Reagenz gebildeten Signals nachgewiesen werden kann.

Alternativ dazu kann auch ein Immunassay-System verwendet werden, worin der tertiäre Komplex mit einem markierten anti-Immunglobulin zur Reaktion gebracht wird und das Reaktionsprodukt anhand seines spezifisch nachweisbaren Signals erkannt werden kann.

Als Marker verwendet man vorzugsweise ein Enzym. Es kann darüber hinaus aber auch Biotin, ein Radioisotop, ein Fluorophor oder jedes beliebige andere für diesen Zweck geeignete und üblicherweise verwendete Reagenz benutzt werden.

Ein weiterer Gegenstand der vorliegenden Erfindung betrifft somit ein Verfahren zum Nachweis des Vorliegens von Phytophthora Antigen in einer besagtes Antigen enthaltenden Probe, das durch die folgenden Verfahrensmassnahmen gekennzeichnet ist:

a) Ausbildung eines binären Komplexes zwischen dem Antigen in der Probe und einem ersten monoklonalen oder polyklonalen Antikörper, der in der Lage ist, mit besagtem Antigen zu reagieren;

b) Ausbildung eines tertiären Komplexes, indem man den binären Komplex mit einem zweiten monoklonalen oder polyklonalen Antikörper in Kontakt bringt, der in der Lage ist, mit besagtem Antigen zu reagieren und

c) Nachweis des Vorliegens eines tertiären Komplexes, aufgrund eines wahrnehmbaren Signals, das von einem analytisch nachweisbaren Reagenz produziert wird; das gegebenenfalls an einem dritten Antikörper konjugiert vorliegen kann;

wobei zumindest einer der Antikörper ein monoklonaler Antikörper ist, der mit mindestens einem pathogenen Stamm aus der Gattung Phytophthora reagiert, der aber im wesentlichen keine Reaktion mit Stämmen aus der Gattung Pythium zeigt und der in der Lage ist Phytophthora in erkranktem Gewebe in einem zwei Antikörper umfassenden Immunoassay nachzuweisen.

Um dem Nachweis zu erleichtern können die verschiedenen Reaktionen vorteilhafterweise in Form eines Kits zur Verfügung gestellt werden.

Der Kit sollte typischerweise einen Carrier beinhalten, der kompartimentiert ist, so dass darin in enger räumlicher Beziehung zueinander angeordnet sind:

(1) ein fester Träger, welcher einen ersten monoklonalen oder polyklonalen Antikörper gebunden enthält, der in der Lage ist einen binären Komplex mit Phytophthora Antigen zu bilden; und

(2) ein Nachweis-System für den binären Komplex bestehend aus einem zweiten monoklonalen oder polyklonalen Antikörper sowie gegebenenfalls einem dritten Antikörper

wobei einer dieser besagten Antikörper ein monoklonaler Antikörper gemäss vorliegender Erfindung ist.

Wie aus der vorangegangenen Diskussion eindeutig hervorgeht, kann es sich dabei entweder um den ersten oder den zweiten Antikörper handeln.

Der zweite Antikörper kann selbst markiert sein oder aber das binäre Nachweissystem enthält einen dritten anti-Immunglobulin Antikörper, der markiert ist und zum Nachweis des tertiären Komplexes, bestehend aus einem ersten Antikörper, dem Antigen, und einem zweiten Antikörper, verwendet werden kann.

Wenn es sich um ein Enzym-Immunassay handelt, dann wird auch noch ein Substrat für das Enzym bereitgestellt.

8

Ein weiterer Gegenstand der vorliegenden Erfindung betrifft somit einen Test-Kit zur immunologischen Diagnose von Phytophthora Infektionen in Pflanzen, der gekennzeichnet ist durch einen Carrier, welcher kompartimentiert ist, so dass darin in enger räumlicher Beziehung zueinander angeordnet sind:

a) ein festen Träger, der einen ersten monoklonalen oder polyklonalen Antikörper gebunden enthält, welcher in der Lage ist mit Phytophthora zu reagieren; und

b) ein Nachweissystem für einen binären Komplex, das einen zweiten monoklonalen oder polyklonalen Antikörper sowie gegebenenfalls einen dritten Antikörper enthält;

wobei zumindest einer der Antikörper ein monoklonaler Antikörper ist, der mit mindestens einem pathogenen Stamm aus der Gattung Phytophthora reagiert, der aber im wesentlichen keine Reaktion mit Stämmen aus der Gattung Pythium zeigt und der in der Lage ist, Phytophthora in erkranktem Gewebe in einem zwei Antikörper umfassenden Immunoassay nachzuweisen.

Die im folgenden aufgeführten Ausführungsbeispiele dienen der näheren Illustrierung der eher allgemein gehaltenen Beschreibung sowie dem besseren Verständnis der vorliegenden Erfindung ohne jedoch den Erfindungsgegenstand in irgendeiner Weise zu limitieren.

Nicht-limitierende Ausführungsbeispiele

Beispiel 1: Verfahren zur Extraktion pilzlicher Proteine

Pilze werden in 50 ml PDB ⌊Potato Dextrose Hohl's medium (Hohl, H.R., Phytopathol. Z., 84: 18-33, 1975)⌋ in 250 ml Flaschen ⌊gewöhnlich verwendet man 2 Liter Phytophthora megasperma f.sp. glycinea, Kaun und Erwin (Pmg)⌋ kultiviert.

Nach einer Woche werden die Pilzkulturen aus dem Medium abgeerntet und zweimal in PBS (Phosphat-gepufferte Salzlösung; pH 7,4) gewaschen.

Die Pilzkulturen werden dann in eine 300 ml Kammer einer DYNO-Mühle, Typ KDL Gewebezerkleinerer (W.A. Bachhofen AG Maschinenfabrik, Basel, Schweiz) überführt, die 240 ml bleifreier Glaskügelchen (IMPANDEX, Maywood, New Jersey, USA) mit einem Durchmesser von 0,5 mm enthält.

Der Kühlmantel der Probenkammer wird mit kaltem Leitungswasser auf 8°C vorgekühlt. Man lässt den Extrakt 5 Minuten bei 3000 rpm absetzen und transferiert dann den Inhalt der Probenkammer in 50 ml Polystyren-Röhrchen und zentrifugiert bei 17,000 rpm (34,540 g) in einer Sorvall RC-5B Kühlzentrifuge unter Verwendung eines SS-34 Rotors.

Der Ueberstand wird anschliessend portioniert und bei -20°C bis zum weiteren Gebrauch tiefgefroren.

Der Gesamt-Proteinanteil der Proben bewegt sich in einem Bereich zwischen 0,5 mg/ml und 2 mg/ml.

Beispiel 2: Monoklonale Antikörper-Produktion

Bei dem hier verwendeten Verfahren handelt es sich um eine Modifikation des bei Köhler und Milstein (1975) sowie des bei Hammerling (1977) beschriebenen Verfahrens.

Die Test-Tiere sind 4 bis 5 Wochen alte weibliche BALB/cJ Mäuse, die vom Jackson Laboratorium in Bar Harbor, ME 04609 bezogen werden.

Die erste Injektion beinhaltet 0,2 ml des Pilzmycels Pmg (Phytophthora megasperma f.sp. glycinea, extrahiert in PBS-Puffer und emulgiert in 0,2 ml Freund's komplettem Adjuvans) und wird durch i.p. Injektion verabreicht.

Die zweite Injektion, die 11 Monate später erfolgt, beinhaltet ebenfalls 0,2 ml des Pilzmycels Pmg (Phytophthora megasperma f.sp. glycinea extrahiert in PBS Puffer und emulgiert mit 0,2 ml Freund's inkomplettem Adjuvans) und wird ebenfalls per i.p. Injektion verabreicht.

Aus dem Schwanz der behandelten Tiere werden 50 µl Mäuseserum gewonnen, das auf positive Pmg Aktivität hin untersucht wird.

Beispiel 3: Isolierung immunkompetenter Milzzellen

7 Tage nach der letzten Injektion wird das Tier durch Genickbruch getötet.

Die Milz wird entfernt und in 20 ml eines "Dulbecco's Modified Eagles' Medium" (DMEM) (Tissue Culture Standards Committee, In Vitro, Vol. 6(2); 63, 1970; Dulbecco R and Freeman G, Virology, 8: 396, 1959) eingebracht.

Die Milz wird auf ein 80 maschiges steriles Gittersieb plaziert, geschnitten und mit DMEM gespült und anschliessend leicht massiert unter Verwendung eines sterilen Kolbens einer 10 cc Einmalspritze.

Während der gesamten Milzzellen Extraktion wird das Gittersieb ständig mit DMEM gespült.

Das gesamte Spülwasser wird in 50 ml Einmal-Zentrifugenröhrchen pipettiert und bei 1200 rpm 10 min. zentrifugiert (die Zentrifugation erfolgt bei Raumtemperatur).

Der Ueberstand wird verworfen und das Zell-Pellet mit 10 ml einer Rote Blutzellen lysierenden Lösung (0,83 % $NH_4Cl$; 0,01 M $KHCO_3$, 0,1 mM EDTA) für einen Zeitraum von 90 Sekunden bei Raumtemperatur gewaschen.

Die Lysierungsreaktion wird durch verdünnen mit 40 ml DMEM gestoppt.

Man lässt die Probe für 3 Minuten stehen und pipettiert anschliessend den Ueberstand in 50 ml Zentrifugenröhrchen.

Nach der Zentrifugation wird das Pellet mit 50 ml DMEM gewaschen und erneut zentrifugiert.

Eine kleine Probe der Milzzellen wird zum Zählen und zur Ueberprüfung der Lebensfähigkeit der Zellen zurückbehalten. Die Gesamtzahl an Milzzellen beträgt $7 \times 10^7$ Zellen/5 ml.

Myeloma Zellen (SP2-O-Ag14 bezogen von der American Type Culture Collection) werden aus einer Kultur in sterile 50 ml Polypropylen-Einmal-Röhrchen (Falcon) transferiert ($7 \times 10^6$ Zellen). Die für die Fusion vorgesehenen Myeloma-Zellen werden zentrifugiert (1200 rpm, 10 Minuten bei Raumtemperatur).

Nach der Zentrifugation wird der Ueberstand in ein sauberes Becherglas gegeben; die Zellen werden mit DMEM gewaschen und erneut zentrifugiert.

In das Röhrchen mit dem gewaschenen Myeloma-Pellet werden die Milzzellen hinzugegeben.

Die Myeloma- und Milzzellen werden dann vorsichtig mit Hilfe einer 10 ml Pipette und eines automatischen Pipettenhebers resuspendiert und 10 Minuten bei 1200 rpm und Raumtemperatur zentrifugiert. Der Ueberstand wird verworfen.

Beispiel 4: Zell-Fusion

Das Fusionsmedium, PEG 1500 (B.M. Bioproducts; cat#783-641) wird auf 37°C vorgewärmt. 1 ml des Fusionsmediums wird tropfenweise in das Röhrchen mit den resuspendierten Myeloma- und Milzzellen zugegeben.

Die letzten 7 Minuten der Fusionsreaktion sind der stufenweisen Verdünnung des PEG mit DMEM vorbehalten.

Am Ende der Verdünnung beträgt das Gesamtvolumen im Röhrchen etwa 30 ml.

Während der gesamten Fusionsperiode wird das Röhrchen leicht bewegt um eine ausreichende Durchmischung des im Röhrchen befindlichen Materials zu gewährleisten.

Das Röhrchen wird dann zentrifugiert (1200 rpm, 10 Minuten bei Raumtemperatur) und der Ueberstand entfernt.

Vorgewärmtes HAT Medium (33 ml) wird in das Röhrchen gegeben und der zelluläre Inhalt mit Hilfe einer 10 ml Pipette resuspendiert.

Die Zellen werden auf insgesamt sieben 96 Loch-Mikrotiterplatten ("Gibcoware cell culture cluster dish") verteilt. In jede Vertiefung der Mikrotiterplatten werden 150 $\mu$l des fusionierten Myeloma/Milz-Materials eingebracht. Die äusseren Vertiefungen werden dann mit HAT-Medium gefüllt. Die Mikrotiter-Platten werden in einen mit einem Wassermantel umgebenen $CO_2$ (7 %) Inkubator bei einer Temperatur von 37°C inkubiert. Die Zellen werden dann alle 4 Tage mit frischem HAT-Medium folgender Zusammensetzung versorgt:

| HAT Medium | | Zusammensetzung |
|---|---|---|
| DMEM (Dulbecco's Modified | | |
| Eagles' Medium) | | 766 ml |
| L-Glutamat | | 10 ml |
| Penicillin/Streptomycin (10'000 Ein- | | 10 ml |
| heiten/100 ml HAT) | | |
| Aminopterin (2 x $10^{-5}$ M Lösung) | | 4 ml |
| Hypoxanthin/Thymidine: | | 10 ml |
| 1 N NaOH Thymidine | 38,8 mg | |
| Hypoxanthin | 136,1 mg | |
| in 100 ml sterilem Wasser | | |
| Hyclone Fötales Rinderserum | | 200 ml |
| cat# A-111-L | | |

Hybridoma Plaques beginnen nach 7 bis 10 Tagen zu erscheinen.

Beispiel 5: Hybridoma-Screening

Diejenigen Hybridomas, die Antikörper gegen pilzliche Pathogene produzieren, können mit Hilfe von vorbereitetem Pilzmaterial von Phytophthora megasperma f.sp. glycinea (Proteinkonzentration 15 µg/ml in PBS-Puffer, siehe oben) in einem ELISA Format identifiziert werden (siehe Roitt et al, Immunology, C.V. Mosby, 1985).

Diejenigen Vertiefungen, die positive Antworten auf die ELISA Tests ergeben, werden einer limitierten Verdünnung unterzogen, so dass reine Stämme von Hybridom-Zellen kultiviert werden können.

Die limitierte Verdünnungsmethode beinhaltet die Kultivierung von periodisch verdünnten Hybridoma-Suspensionen.

Jede Verdünnungsstufe wird in 6 bis 12 Vertiefungen einer 96-Loch Kulturplatte etabliert. Diese werden dann erneut auf eine spezifische Antikörper Aktivität gegenüber pilzlichem Protein hin untersucht.

Der Inhalt positiver Vertiefungen wird dann für die Massenkultivierung in 20 ml Kulturfläschchen transferiert.

5.1 Screening Protokoll

ELISA-Glutaraldehyd-Verfahren

20 µl eines Glutaraldehyd Puffers wird in jede Vertiefung der Kulturplatten (Costar, Enzyme-Immuno-Assay Platten, Bio Rad, Richmond, Calf., USA) gegeben und 3 Stunden bei 55°C inkubiert.

Die Platte wird auf Raumtemperatur abgekühlt und der verbleibende Puffer verworfen. Die Platten werden 4 mal mit destilliertem Wasser gewaschen. 200 µl Antigen verdünnt in PBS, pH 7,2 (Antigen Konzentration 10 µg/ml) wird auf die einzelnen Vertiefungen verteilt und 24 Stunden bei 4°C inkubiert. Die verbleibende Lösung wird verworfen und die Platte 8 mal mit PBS gewaschen. 200 µl (Mono)ethanolamin Lösung wird dann auf jede Vertiefung verteilt und weitere 20 Stunden bei 4°C inkubiert.

Die verbleibende Lösung wird verworfen und die Platte 8 mal mit PBS gewaschen. 100 µl des Ueberstandes (antikörperhaltiges Exudat, welches von den Hybridoma-Zellen ins Medium abgegeben wird) wird in jede Vertiefung gegeben und 2 Stunden bei 33°C bei hoher Luftfeuchtigkeit inkubiert.

Die verbleibende Lösung wird verworfen und die Platte 8 mal mit PBS gewaschen.

100 µl KPL biotinyliertes Ziegen anti-Maus IgG oder IgM (Kirkegaard and Perry Laboratories Inc., Maryland, USA) [Verdünnung 1:2,500 in 1 % BSA (Bovine Serum Albumin); Verdünnungsmittel PBS] wird dann zu jeder Vertiefung hinzugegeben. Diese werden bei 37°C bei hoher Luftfeuchtigkeit 0,5 Stunden inkubiert, die Lösung wird anschliessend verworfen und die Platte 8 mal mit PBS gewaschen.

100 $\mu$l KPL Streptavidin (Peroxidase-Konjugat; Kirkegaard and Perry Laboratories Inc., Maryland, USA) konjugiert an Peroxidase Enzym wird in jede Vertiefung gegeben und bei 37°C bei hoher Luftfeuchtigkeit für 0,5 Stunden inkubiert.

Die überstehende Lösung wird verworfen und die Platte 8 mal mit PBS gewaschen. 200 $\mu$l OPD Substrat wird in jede Vertiefung der Platte gegeben und diese 0,5 Stunden bei Raumtemperatur inkubiert.

Anschliessend wird die Absorptionsfähigkeit im Rotbereich bei 405 nm bestimmt.

### 5.2 Benötigte Lösungen

1. Glutaraldehyd-Puffer 0,1 % Glutaraldehyd in 0,1 M Carbonat-Puffer. Der Carbonat-Puffer, pH 9,0, ist zusammengesetzt aus: 1,59 g $Na_2CO_3$ und 2,93 g $NaHCO_3$ pro Liter destilliertes Wasser.
2. PBS: 8,0 g NaCl, 0,2 g $KH_2PO_4$, 1,15 g $Na_2HPO_4$ (wasserfrei), 0,2 g KCl, pro Liter destilliertes Wasser, pH 7,4.
3. (Mono)ethanolamin Lösung: 1 mg/ml Lösung (1 g/Liter destilliertes Wasser).
4. Konjugat (KPL): Verdünnung 1:2500; 1 % BSA ("Bovine Serum Albumin")-Verdünnungsmittel PBS.
5. Natrium Citrat Puffer: 7,1 g $Na_2HPO_4$ (dibasische Lösung) in 500 ml destilliertem Wasser; 9,6 g Zitronensäure in 500 ml destilliertem Wasser. Zitronensäure Lösung wird zu der dibasischen Lösung solange hinzugegeben, bis ein pH von 4,5 erreicht ist.
6. OPD Substrat: 8,0 mg OPD und 20,0 mg Harnstoff Peroxid in 20 ml Natrium-Citrat-Puffer (pH 4.5).

### 5.3 Screening-Ergebnisse für die Ueberstände der Zellinie PH4830 (ATCC HB9353)

Der folgende Test wurde an mit Glutaraldehyd präparierten Platten, die eine Reihe verschiedener Antigene enthalten, durchgeführt. Das für das Screening verwendete Konjugat (KPL) besteht aus Ziegen anti-Maus-IgG-Biotin-Streptavidin-Peroxidase. Eine Absorption von weniger als 0,2 wird als im wesentlichen keine Reaktion zeigend angesehen.

Tabelle 2

| Abkürzung | Phytophthora Spezies | Absorption |
|---|---|---|
| Ph5 | Phytophthora | 0.73 |
| Phc2 | P. citrophthora | 0.44 |
| Phc11 | P. citricola | 0.59 |
| Phcm1 | P. cambivora | 0.43 |
| Phcn1 | P. cinnamomi | 0.20 |
| Phcp1 | P. capsici | 0.71 |
| Phcr-3 | P. cryptogea | 0.55 |
| Phd-1 | P. dreschsleri | 0.41 |
| Phe-1 | P. erythroseptica | 0.51 |
| Phn-1 | P. nicotianae | 0.37 |
| Php-3 | P. parasitica | 0.47 |
| | | |
| Pmeg-19 | P. megasperma | 0.59 |
| Pmgr4-1 | P. megasperma f.sp. glycinea | 0.41 |
| Pmgr2-1 | P. megasperma f.sp. glycinea | 0.60 |
| Pmm-2 | P. megasperma f.sp. medicaginis | 0.79 |
| Ppn-14 | P. parasitica var. nicotianae | 0.38 |
| Pmeg-2 | P. megasperma | 0.34 |
| Ppn-14 | P. parasitica nicotianae | 0.41 |
| Pmeg-2 | P. megasperma | 0.46 |

| Abkürzung | Pythium Spezies | Absorption |
|---|---|---|
| Pa-1 | Pythium aphanidermatum | 0.05 |
| Pa4 | P. aphanidermatum | 0.01 |
| Pa1 | P. aphanidermatum | 0.01 |
| Pa6 | P. aphanidermatum | 0.10 |
| Pa15 | P. aphanidermatum | 0.04 |
| Pa1 | P. aphanidermatum | 0.07 |
| | | |
| Pg1 | P. graminicola | 0.05 |
| Pc1 | P. coloratum | 0.06 |
| Pm1 | P. mamillatum | 0.04 |
| Pi4 | P. irregulare | 0.02 |
| Pv1 | P. vexans | 0.16 |
| Pd1 | P. dissotochum | 0.02 |

| Pval | P. vanterpoolii | 0.01 |
| Pt2 | P. torulosum | 0.10 |
| Pt5 | P. torulosum | 0.01 |
| | | |
| Pul | P. ultimum | 0.06 |
| Pmyl | P. myriotylum | 0.00 |
| Psvl | P. sylvaticum | 0.06 |
| Pil | P. irregulare | 0.02 |
| Pusl | P. ultimum var. sporangiiforum | 0.03 |
| Prl | P. rostratum | 0.02 |
| Psl | P. saliingosperum | 0.05 |

| Abkürzung | andere Pilz Spezies | Absorption |
| Rs-16a | Rhizoctonia solani | 0.02 |
| Sh-1 | Sclerotinia homoeocarpa | 0.01 |
| Rc-1 | Rhizoctonia cerealis | 0.01 |
| | | |
| PBS | Phosphat-gepufferte Salzlösung | 0.00 |

Beispiel 6: Affinitäts-Test

Die folgenden Verfahrensmassnahmen werden durchgeführt, um die Affinität der produzierten monoklonalen Antikörper zu bestimmen.

Der zu testende Antikörper wird in einem Masse verdünnt, dass bei einer Antikörper Titration schätzungsweise ein Wert von 1,5 O.D. erreicht wird.

Die Verdünnungslösung hat die folgende Zusammensetzung:

Antikörper Verdünnungspuffer: pH 7,2

| Bestandteil | Konzentration |
| --- | --- |
| $Na_2HPO_4$ | 2,19 g/l |
| $NaH_2PO_4$ | 0,56 g/l |
| NaCl | 8,76 g/l |
| Thimerosal | 0,1 g/l |
| BSA ("Bovine Serum Albumin") | 1,0 g/l |

50 ml einer bereits gebrauchsfertigen vorverdünnten Standardlösung wird mit Hilfe einer Repetitionspipette in jede Vertiefung gegeben. Der Inhalt der Vertiefungen wird 10 Minuten unter Schütteln bei Raumtemperatur inkubiert. Die Vertiefungen werden anschliessend 5 mal mit einer Waschlösung der folgenden Zusammensetzung gewaschen:

| Bestandteil | Konzentration |
| --- | --- |
| Tris | 2,42 g/l |
| NaCl | 8,76 g/l |
| Thimerosal | 0,10 g/l |
| Tween 80 | 5,00 g/l |
| HCl (1.0N) | ca. 14,3 ml/l |
| (HCl Zugabe bis ein pH von 7,8 erreicht ist) | |

Bei dem verwendeten Konjugat handelt es sich um ein 1:500 "Dakopatts" (Dakopatts A/S; Dänemark) anti-Maus-IgG-Meerrettichperoxidase Konjugat; dies wird in einer Menge von 100 $\mu$l in jede Vertiefung gegeben und unter Schütteln 10 Minuten bei Raumtemperatur inkubiert. Die Vertiefungen werden erneut gewaschen und anschliessend wird 100 $\mu$l Substrat zu jeder Vertiefung zugegeben.

Das Substrat ist in 15 mg/ml einer 2,2'-Azinobis(3)-Ethylbenzthiazolinsulfonsäure) (Diammoniumsalz) (ABTS) Stammlösung enthalten, 1:25 verdünnt mit einem Citrat-Peroxidase Puffer der folgenden Zusammensetzung:

Citrat-Peroxidase Puffer: pH 4,0

| Bestandteil | Konzentration |
|---|---|
| Zitronensäure $H_2O$<br>Einstellen des pH auf 4.0 mit 1.0N NaOH<br>Auffüllen auf ein Gesamtvolumen von 100 ml mit $H_2O$ vor der Zugabe von $H_2O_2$<br>$H_2O_2$ (30 %) | 2,3 g/85 ml<br><br><br>50 $\mu$l/100 ml |

Nach der Zugabe des Substrats werden die Platten abermals für 10 Min. bei Raumtemperatur unter Schütteln inkubiert. Eine Stop-Lösung (50 ml 15 % Natriumfluorid) wird dann zu jeder Vertiefung zugegeben und 10 Sekunden vermischt.

Anschliessend wird die Absorptionsfähigkeit zwischen 405 nm und 415 nm bestimmt.

Eine Dosis-abhängige Wirkungskurve wird anhand eines semi-logarithmischen Schaubild erstellt und diejenige Antigen-Konzentratration mittels Extrapolation bestimmt, die eine 50 %ige Reduktion der maximalen Farbentwicklung verursacht. Dieser Wert wird als ein relatives Mass für die Affinität betrachtet.

Dieses Verfahren wird mit den erfindungsgemäss hergestellten Antikörpern durchgeführt. Als ein Beispiel einer kalkulierten Affinität für die erfindungsgemässen Antikörper sei Antikörper No. 4830 genannt mit einer Affinität von ca. 4,3 $\mu$g/ml Antigen bei 50 % des maximalen OD-Wertes ohne Antigen.

Beispiel 7: Subklonierungsmessnahmen

Diejenigen Vertiefungen, die eine positive Antwort in den ELISA Tests erbringen, werden einer limitierten Verdünnung unterworfen, so dass reine Stämme von Hybridoma Zellen kultiviert werden können.

Das limitierte Verdünnungsverfahren beinhaltet die Kultivierung periodisch verdünnter Hybridoma-Suspensionen.

Jede Verdünnungsreihe wird in 6 bis 12 Vertiefungen einer 96-Loch-Kulturplatte etabliert. Diese Vertiefungen werden dann erneut auf eine spezifische Antikörperaktivität gegen pilzliches Protein hin untersucht. Der Inhalt positiver Vertiefungen wird dann für die Massenkultivierung in 20 ml Kulturflaschen überführt.

7.1 Charakterisierung von Klon # PH4830

Klon # PH4830 sezerniert Antikörper der IgG2b Subklasse gegen Phytophthora megasperma f.sp. glycinea.

7.2 Hinterlegung

Eine Hinterlegung einer biologisch reinen Kultur der folgenden Hybridoma-Zellinie wurde bei der "American Type Culture Collection, 12301 Parklawn Drive, Rockville, Maryland am 12. März 1987 vorgenommen. Die angegebene Eingangsnummer wurde nach der erfolgreichen Durchführung des Lebensfähigkeitstests zuerkannt und die geforderten Gebüren wurden bezahlt.

Sollte die Probe ihre Lebensfähigkeit einbüssen oder aber versehentlich zerstört werden, so wird sie durch eine lebensfähige Kultur mit der gleichen taxonomischen Beschreibung ersetzt werden.

| Hybridoma | ATCC Nr. |
|---|---|
| Balbc Maus/SP2 Myelom #PH4830 | HD9353 |

Nachweis von Pilz-Pathogenen und Kits

Die vorliegende Erfindung umfasst auch die Verwendung der zuvor beschriebenen Antikörper in einen System zum Nachweis von Phytophthora Infektionen in erkranktem Gewebe. Dementsprechend wird eine Probe pflanzlichen Materials, das im Verdacht steht den Organismus zu enthalten, mit einem ersten Antikörper, der spezifisch mit einer antigenen Determinanten des nachzuweisenden Organismus reagiert, in Kontakt gebracht. Vorzugsweise handelt es sich bei besagtem Antikörper um einen an einem festen Träger, wie z.B. an den Wänden einer Mikrotiterplatte immobilisierten Antikörper.

Der Antikörper kann ein monoklonaler Antikörper oder aber ein Bestandteil eines polyklonalen Serums sein, das mit Phytophthora reagiert.

Nach Entfernen des nicht umgesetzten Materials durch Auswaschen wird der verbleibende binäre Komplex (Antigen-Antikörper-Komplex) mit einem monoklonalen oder polyklonalen Antikörper in Kontakt gebracht, der spezifisch mit dem nachzuweisenden Antigen reagiert.

Durch den Kontakt des immobilisierten binären Komplexes mit dem zweiten monoklonalen Antikörper entsteht ein tertiärer Komplex (Antikörper-Antigen-Antikörper). Zumindest bei einem der Antikörper, die am Aufbau des tertiären Komplexes beteiligt sind, handelt es sich um einen erfindungsgemässen Antikörper. Nach dem Entfernen all derjenigen sekundären Antikörper, die nicht an den sekundären Komplex gebunden haben, durch Auswaschen, kann der entstandene tertiäre Komplex mit Hilfe einer Reihe von verschiedenen analytischen Techniken nachgewiesen werden. Der zweite Antikörper kann z.B. direkt mit einem analytisch bestimmbaren Reagenz markiert werden, so dass der gebildete tertiäre Komplex dann anhand eines durch dieses Reagenz gebildeten Signals nachgewiesen werden kann.

Alternativ dazu kann auch ein Immunassay-System verwendet werden, worin der tertiäre Komplex mit einem markierten anti-Immunglobulin zur Reaktion gebracht wird und das Reaktionsprodukt anhand seines spezifisch nachweisbaren Signals erkannt werden kann.

Als Marker verwendet man vorzugsweise ein Enzym. Es kann darüber hinaus aber auch Biotin, ein Radioisotop, ein Fluorophor oder jedes beliebige andere für diesen Zweck geeignete und üblicherweise verwendete Reagenz benutzt werden.

Um den Nachweis zu erleichtern können die verschiedenen Reaktionen vorteilhafterweise in Form eines Kits zur Verfügung gestellt werden.

Der Kit sollte typischerweise einen Carrier beinhalten, der kompartimentiert ist, so dass darin in enger räumlicher Beziehung zueinander angeordnet sind:

(1) ein fester Träger, welcher einen ersten monoklonalen oder polyklonalen Antikörper gebunden enthält, der in der Lage ist einen binären Komplex mit Phytophthora Antigen zu bilden; und

(2) ein Nachweis-System für den binären Komplex bestehend aus einem zweiten monoklonalen oder polyklonalen Antikörper sowie gegebenenfalls einem dritten Antikörper

wobei einer dieser besagten Antikörper ein monoklonaler Antikörper gemäss vorliegender Erfindung ist.

Wie aus der vorangegangenen Diskussion eindeutig hervorgeht, kann es sich dabei entweder um den ersten oder den zweiten Antikörper handeln.

Der zweite Antikörper kann selbst markiert sein oder aber das binäre Nachweissystem enthält einen dritten anti-Immunglobulin Antikörper, der markiert ist und zum Nachweis des tertiären Komplexes, bestehend aus einem ersten Antikörper, dem Antigen, und einem zweiten Antikörper, verwendet werden kann.

Wenn es sich um ein Enzym-Immunassay handelt, dann wird auch noch ein Substrat für das Enzym bereitgestellt.

Die im folgenden aufgeführten Ausführungsbeispiele dienen der näheren Illustrierung der eher allgemein gehaltenen Beschreibung sowie dem besseren Verständnis der vorliegenden Erfindung ohne jedoch den Erfindungsgegenstand in irgendeiner Weise zu limitieren.

Ein spezifisches Beispiel für das Einbringen von erfindungsgemässen Antikörpern in ein ELISA-Format, das dazu dient die Brauchbarkeit für die Anwendung in einem diagnostischen Test-Kit zu demonstrieren, kann auf die folgende Art und Weise durchgeführt werden:

Ascites Flüssigkeit von Mäusen wird auf eine Aktivität gegenüber mit Antigen-sensitivierten "Mikrowell" Modulen hin untersucht unter Verwendung eines Antikörper Screening-Verfahrens.

Verdünnungen von $10^{-4}$ ergeben Absorptionswerte, die ausserhalb des Messbereiches liegen ($\geqq 2.0$), bei

Verdünnungen von $10^{-5}$ dagegen erhält man Werte von 0,5 OD bei 415 nm.

Die Antikörper werden aus der Ascites-Flüssigkeit mit Hilfe einer Protein A Affinitätschromatographie nach folgendem Schema gereinigt:

16

Chromatographie-Parameter:

1. Gel - Pharmacia CL4B Protein A (6,0 ml)
2. Tris/HCl Aequilibrieruns-/Bindungs Puffer pH 8,6
3. Acetat Elutionspuffer pH 4,3
4. Glycin/HCl Regenerationspuffer pH 2,3
5. Pumpengeschwindigkeit 12 (ca. 1 ml/min.)
6. Beladung mit 2,5 ml ungereinigter Ascites-Flüssigkeit
7. Papiergeschwindigkeit 5 mm/min.
8. 0,2 O.D. Vollauschlag.

Die Ascites Flüssigkeit wird über eine Protein A Säule gegeben und die IgG-Fraktion aus der Säule bei einem pH von 4,3 eluiert.

Die Aktivität des gereinigten Antikörpers wird mit Hilfe eines einfachen Antikörper-Screening-Verfahrens bestimmt.

Der gereinigte monoklonale Antikörper wird an Meerrettichperoxidase konjugiert mit Hilfe einer Perjodat Oxidation der Karbohydrat-Gruppen des Enzyms unter Ausbildung aktiver Aldehyd-Gruppen, die sich mit den Amino-Gruppen des Antikörpers verbinden.

Das Antikörper-Enzym Konjugat wird an mit Antigen sensitivierten "Multiwell"-Platten getestet.

Für Konjugate mit einer Konzentration von 2,1 $\mu$g IgG/ml und 0,21 $\mu$g IgG/ml erhält man überschiessende Absorptionswerte (2,0+). Bei Verwendung von Konjugaten in einer Konzentration von 0,021 $\mu$g IgG/ml beträgt dagegen der Absorptionswert 0,369.

Der Enzym-konjugierte Antikörper wird in einem doppeltem Antikörper Sandwich ELISA Assay in Verbindung mit Multiwell-Platten verwendet, die zuvor mit über eine Affinitätschromatographie gereinigten polyklonalen Antikörpern aus dem Schaf sensitiviert wurden.

Der verwendete polyklonale Antikörper wird durch Immunisierung von Schafen gewonnen und zwar mit der gleichen Antikörperpräparation die auch für die Herstellung monoklonaler Antikörper verwendet wurde.

Die Anfangsdosis beträgt 4 ml Antigen emulgiert in 4 ml Adjuvanz. Diese wird entlang einer Seite des mittleren Rückenbereich sowie in die Flanken injiziert.

Nach einer Ruheperiode von 30 Tagen erfolgt eine 2. Dosierung von 2 ml Antigen plus 2 ml Adjuvanz, in diejenige Rückenpartie die gegenüber derjenigen der ersten Injektion liegt.

7 bis 10 Tage nach der Anfangsdosierung wird Blut entnommen und der gesamte Zyklus (Injektion und Blutentnahme) wiederholt.

Das ungereinigte polyklonale Serum zeigt gewöhnlich eine Kreuzreaktion mit Pythium, kann aber gegen andere nicht-Zielorganismen gescreent und durch Antigen-Affinitätschromatographie gereinigt werden.

Zusätzlich zu der Ueberprüfung der erfindungsgemässen Antikörper, wird das zuvor beschriebene Verfahren dazu verwendet, eine ganze Reihe von monoklonalen Antikörpern zu testen, die bereits früher in der Literatur beschrieben wurden (z.B. Ayers, supra). Diese Tests werden an Proben von Phytophthorainfizierten und von gesunden Sojabohnen durchgeführt, sowie an abgekochten und nicht abgekochten reinen Kulturen des Pathogens.

Von allen bekannten Antikörpern, die getestet wurden, führte keiner innerhalb einer kurzen Zeitspanne von maximal 20-30 Minuten zu einer positiven Reaktion mit erkranktem Gewebe und erweist sich damit als ungeeignet für eine Verwendung in einem Diagnose-Test-Kit.

Die Ergebnisse des Assays mit Antikörper 4830 sind in Tabelle 3 wiedergegeben.

Tabelle 3

Freiland-Proben von Sojabohnen-Pflanzen, sowohl mit als auch ohne Symptome einer Phytophthora Wurzel- und Stengel-Fäule werden extrahiert und in einem zweifachen Antikörper Immunoassay unter Verwendung des Antikörper-4830-Peroxidase Konjugats getestet. Eine Zusammenfassung der Ergebnisse zeigt die folgende Zusammenstellung:

Stengel mit Symptomen

| | |
|---|---|
| #127 | 0,33 |
| #132 | 0,21 |
| #146 | 2,00 |

Wurzeln mit Symptomen

| | |
|---|---|
| #127 | 0,19 |
| #132 | 0,75 |
| #146 | 2,00 |

Stengel ohne Symptome

| | |
|---|---|
| #161 | ,002 |
| #149 | 000 |
| #158 | 000 |

Wurzeln ohne Symptome

| | |
|---|---|
| #161 | 008 |
| #149 | 000 |
| #158 | 000 |

**Patentansprüche**
**Patentansprüche für folgende Vertragsstaaten : AT, BE, CH, LI, DE, FR, GB, GR, IT, LU, NL, SE**

1. Ein Hybridoma, welche einen monoklonalen Antikörper produziert, der mit mindestens einem pathogenen Stamm aus der Gattung Phytophthora reagiert, der aber im wesentlichen keine Reaktion mit Stämmen aus der Gattung Pythium zeigt und der in der Lage ist, Phytophthora in erkranktem Gewebe in einem zwei Antikörper umfassenden Immunassay nachzuweisen.

2. Hybridoma gemäss Anspruch 1, dadurch gekennzeichnet, dass besagter monoklonaler Antikörper zur IgG2b Subklasse gehört.

3. Ein monoklonaler Antikörper der mit mindestens einem pathogenen Stamm aus der Gattung Phytophthora reagiert, der aber im wesentlichen keine Reaktion mit Stämmen aus der Gattung Pythium zeigt und der in der Lage ist, Phytophthora in erkranktem Gewebe in einem zwei Antikörpor umfassenden Immunassay nachzuweisen.

4. Der Antikörper gemäss Anspruch 3, dadurch gekennzeichnet, dass er zur IgG2b Subklasse gehört.

5. Ein monoklonaler Antikörper hergestellt von einem Hybridoma gemäss Anspruch 1 oder 2.

6. Verfahren zur Herstellung eines Hybridoma gemäss Anspruch 1, dadurch gekennzeichnet, dass man
   a) eine immunkompetente Zelle aus einem zuvor immunisierten Donor-Tier isoliert
   b) mit einer zur kontinuierlichen Zellteilung befähigten Tumorzellinie fusioniert und
   c) das entstehende Fusionsprodukt isoliert.

7. Verfahren gemäss Anspruch 6, dadurch gekennzeichnet, dass es sich bei besagter immunkompetenter Zelle um eine Milzzelle handelt.

8. Verfahren gemäss Anspruch 6, dadurch gekennzeichnet, dass es sich bei besagter Tumorzellinie um eine Myelomazellinie handelt, die selbst keine monoklonalen Antikörper produziert.

9. Verfahren gemäss Anspruch 8, dadurch gekennzeichnet, dass es sich um die Myelomazellinie SP2-O-Ag14 oder X63-Ag8.653 handelt.

10. Verfahren zur Herstellung eines monoklonalen Antikörpers gemäss Anspruch 3, dadurch gekennzeichnet, dass man ein Hybridoma, welches einen monoklonalen Antikörper produziert, der mit mindestens einem pathogenen Stamm aus der Gattung Phytophthora reagiert, der aber im wesentlichen keine Reaktion mit Stämmen aus der Gattung Pythium zeigt und der in der Lage ist, Phytophthora in erkranktem Gewebe in einem zwei Antikörper umfassenden Immunassay nachzuweisen, in vitro oder in vivo massenkultiviert.

11. Verfahren gemäss Anspruch 10, dadurch gekennzeichnet, dass man ein Hybridoma gemäss einem der Ansprüche 2 bis 5 massenkultiviert.

12. Verfahren zum Nachweis des Vorliegens von Phytophthora Antigen in einer besagtes Antigen enthaltenden Probe, das durch die folgenden Verfahrensmassnahmen gekennzeichnet ist:
    a) Ausbildung eines binären Komplexes zwischen dem Antigen in der Probe und einem ersten monoklonalen oder polyklonalen Antikörper, der in der Lage ist, mit besagtem Antigen zu reagieren;
    b) Ausbildung eines tertiären Komplexes, indem man den binären Komplex mit einem zweiten monoklonalen oder polyklonalen Antikörper in Kontakt bringt, der in der Lage ist, mit besagtem Antigen zu reagieren und
    c) Nachweis des Vorliegens eines tertiären Komplexes, aufgrund eines wahrnehmbaren Signals, das von einem analytisch nachweisbaren Reagenz produziert wird; das gegebenenfalls an einem dritten Antikörper konjugiert vorliegen kann;
    wobei zumindest einer der Antikörper ein monoklonaler Antikörper ist, der mit mindestens einem pathogenen Stamm aus der Gattung Phytophthora reagiert, der aber im wesentlichen keine Reaktion mit Stämmen aus der Gattung Pythium zeigt und der in der Lage ist Phytophthora in erkranktem Gewebe in einem zwei Antikörper umfassenden Immunoassay nachzuweisen.

13. Verfahren gemäss Anspruch 12, dadurch gekennzeichnet, dass das nachweisbare Reagenz an den zweiten Antikörper konjugiert vorliegt.

14. Verfahren gemäss Anspruch 12, dadurch gekennzeichnet, dass das nachweisbare Reagenz an einem dritten, anti-Immunoglobulin Antikörper konjugiert vorliegt, der mit dem tertiären Komplex in Kontakt gebracht wird.

15. Verfahren gemäss Anspruch 12, dadurch gekennzeichnet, dass es sich bei besagtem Reagenz um ein Enzym, ein Radioisotop, ein Fluorophor oder um Biotin handelt.

16. Verfahren gemäss Anspruch 14, dadurch gekennzeichnet, dass es sich bei besagtem Reagenz um ein Enzym, ein Radioisotop, ein Fluorophor oder um Biotin handelt.

17. Verfahren gemäss Anspruch 12, dadurch gekennzeichnet, dass einer der Antikörper an einen festen Träger immobilisiert vorliegt.

**18.** Ein Test-Kit zur immunologischen Diagnose von Phytophthora Infektionen in Pflanzen, der gekennzeichnet ist durch einen Carrier, welcher kompartimentiert ist, so dass darin in enger räumlicher Beziehung zueinander angeordnet sind:

a) ein festen Träger, der einen ersten monoklonalen oder polyklonalen Antikörper gebunden enthält, welcher in der Lage ist mit Phytophthora zu reagieren; und

b) ein Nachweissystem für einen binären Komplex, das einen zweiten monoklonalen oder polyklonalen Antikörper sowie gegebenenfalls einen dritten Antikörper enthält;

wobei zumindest einer der Antikörper ein monoklonaler Antikörper ist, der mit mindestens einem pathogenen Stamm aus der Gattung Phytophthora reagiert, der aber im wesentlichen keine Reaktion mit Stämmen aus der Gattung Pythium zeigt und der in der Lage ist, Phytophthora in erkranktem Gewebe in einem zwei Antikörper umfassenden Immunoassay nachzuweisen.

**19.** Der Test-Kit gemäss Anspruch 18, dadurch gekennzeichnet, dass der zweite Antikörper konjugiert ist an ein analytisch nachweisbares Reagenz ausgewählt aus der Gruppe bestehend aus einem Enzym, einem Radioisotop, einem Fluorophor oder Biotin.

**20.** Test-Kit gemäss Anspruchs 18, dadurch gekennzeichnet, dass der Kit einen dritten anti-Immunoglobulin Antikörper enthält, welcher konjugiert ist an ein analytisch nachweisbares Reagenz ausgewählt aus der Gruppe bestehend aus einem Enzym, einem Radioisotop, einen Fluorophor oder Biotin.

**Patentansprüche für folgenden Vertragsstaat : ES**

**1.** Verfahren zur Herstellung eines Hybridoma, welches einen monoklonalen Antikörper produziert, der mit mindestens einem pathogenen Stamm aus der Gattung Phytophthora reagiert, der aber im wesentlichen keine Reaktion mit Stämmen aus der Gattung Pythium zeigt und der in der Lage ist, Phytophthora in erkranktem Gewebe in einem zwei Antikörper umfassenden Immunassay nachzuweisen, dadurch gekennzeichnet, dass man

a) eine immunkompetente Zelle aus einem zuvor immunisierten Donor-Tier isoliert;

b) mit einer zur kontinuierlichen Zellteilung befähigten Tumorzellinie fusioniert; und

c) das entstehende Fusionsprodukt isoliert.

**2.** Verfahren gemäss Anspruch 1, dadurch gekennzeichnet, dass es sich bei besagter immunkompetenter Zelle um eine Milzzelle handelt.

**3.** Verfahren gemäss Anspruch 1, dadurch gekennzeichnet, dass es sich bei besagter Tumorzellinie um eine Myelomazellinie handelt, die selbst keine monoklonalen Antikörper produziert.

**4.** Verfahren gemäss Anspruch 3, dadurch gekennzeichnet, dass es sich um die Myelomazellinie SP2-O-Ag14 oder X63-Ag8.653 handelt.

**5.** Verfahren gemäss Anspruch 1, dadurch gekennzeichnet, dass besagtes Hybridoma einen monoklonalen Antikörper produziert, der zur IgG2b Subklasse gehört.

**6.** Verfahren zur Herstellung eines monoklonalen Antikörpers, der mit mindestens einem pathogenen Stamm aus der Gattung Phytophthora reagiert, der aber im wesentlichen keine Reaktion mit Stämmen aus der Gattung Pythium zeigt und der in der Lage ist, Phytophthora in erkranktem Gewebe in einem zwei Antikörper umfassenden Immunassay nachzuweisen, dadurch gekennzeichnet, dass man ein Hybridoma gemäss einem der Ansprüche 1 bis 5 in vitro oder in vivo massenkultiviert.

**7.** Verfahren gemäss Anspruch 6, dadurch gekennzeichnet, dass besagter monoklonaler Antikörper zur IgG2b Subklasse gehört.

**8.** Verfahren zum Nachweis des Vorliegens von Phytophthora Antigen in einer besagtes Antigen enthaltenden Probe, das durch die folgenden Verfahrensmassnahmen gekennzeichnet ist:

a) Ausbildung eines binären Komplexes zwischen dem Antigen in der Probe und einem ersten monoklonalen oder polyklonalen Antikörper, der in der Lage ist, mit besagtem Antigen zu reagieren;

b) Ausbildung eines tertiären Komplexes, indem man den binären Komplex mit einem zweiten monoklonalen oder polyklonalen Antikörper in Kontakt bringt, der in der Lage ist, mit besagtem Antigen zu reagieren und

c) Nachweis desVorliegens eines tertiären Komplexes, aufgrund eines wahrmehmbaren Signals, das von einem analytisch nachweisbaren Reagenz produziert wird, das gegebenenfalls an einem dritten Antikörper konjugiert vorliegen kann;

wobei zumindest einer der Antikörper ein monoklonaler Antikörper ist, der mit mindestens einem pathogenen Stamm aus der Gattung Phytophthora reagiert, der aber im wesentlichen keine Reaktion mit Stämmen aus der Gattung Pythium zeigt und der in der Lage ist, Phytophthora in erkranktem Gewebe in einem zwei Antikörper umfassenden Immunassay nachzuweisen.

9. Verfahren gemäss Anspruch 8, dadurch gekennzeichnet, dass das nachweisbare Reagenz an den zweiten Antikörper konjugiert vorliegt.

10. Verfahren gemäss Anspruch 8, dadurch gekennzeichnet, dass das nachweisbare Reagenz an einem dritten, anti-Immunoglobulin Antikörper konjugiert vorliegt, der mit dem tertiären Komplex in Kontakt gebracht wird.

11. Verfahren gemäss Anspruch 8, dadurch gekennzeichnet, dass es sich bei besagtem Reagenz um ein Enzym, eine Radioisotop, ein Fluorophor oder um Biotin handelt.

12. Verfahren gemäss Anspruch 10, dadurch gekennzeichnet, dass es sich bei besagtem Reagenz um ein Enzym, eine Radioisotop, ein Fluorophor oder um Biotin handelt.

13. Verfahren gemäss Anspruch 10, dadurch gekennzeichnet, dass einer der Antikörper an einen festen Träger immobilisiert vorliegt.

14. Mittel zur immunologischen Diagnose von Phytophthora Infektionen in Pflanzen in Form eines gebrauchsfertigen Test-Kits, der gekennzeichnet ist durch einen Carrier, welcher kompartimentiert ist, so dass darin in enger räumlicher Beziehung zueinander angeordnet sind:

a) ein fester Träger, der einen ersten monoklonalen Antikörper oder polyklonalen Antikörper gebunden enthält, welcher in der Lage ist mit Phytophthora zu reagieren; und

b) ein Nachweissystem für einen binären Komplex, das einen zweiten monoklonalen oder polyklonalen Antikörper sowie gegebenenfalls einen dritten Antikörper enthält;

wobei zumindest einer der Antikörper ein monoklonaler Antikörper ist, der mit mindestens einem pathogenen Stamm aus der Gattung Phytophthora reagiert, der aber im wesentlichen keine Reaktion mit Stämmen aus der Gattung Pythium zeigt und der in der Lage ist, Phytophthora in erkranktem Gewebe in einem zwei Antikörper umfassenden Immunassay nachzuweisen.

15. Mittel gemäss Anspruch 14, dadurch gekennzeichnet, dass der zweite Antikörper konjugiert ist an ein analytisch nachweisbares Reagenz ausgewählt aus der Gruppe bestehend aus einem Enzym, einem Radioisotop, einem Fluorophor oder Biotin.

16. Mittel gemäss Anspruch 14, dadurch gekennzeichnet, dass der Kit einen dritten anti-Immunoglobulin Antikörper enthält, welcher konjugiert ist an ein analytisch nachweisbares Reagenz ausgewählt aus der Gruppe bestehend aus einem Enzym, einem Radioisotop, einem Fluorophor oder Biotin.

**Claims**
**Claims for the following Contracting States : AT, BE, CH, LI, DE, FR, GB, GR, IT, LU, NL, SE**

1. A hybridoma which produces a monoclonal antibody which reacts with at least one pathogenic strain of the genus Phytophthora but which exhibits substantially no reaction with strains of the genus Pythium and is capable of detecting Phytophthora in diseased tissue in a double-antibody immunoassay.

2. A hybridoma according to claim 1, wherein said monoclonal antibody belongs to the IgG2b subclass.

3. A monoclonal antibody which reacts with at least one pathogenic strain of the genus Phytophthora but which exhibits substantially no reaction with strains of the genus Pythium and is capable of detecting Phytophthora in diseased tissue in a double-antibody immunoassay.

**4.** The antibody according to claim 3, which belongs to the IgG2b subclass.

**5.** A monoclonal antibody produced by a hybridoma according to claim 1 or 2.

**6.** A method of producing a hybridoma according to claim 1, which comprises
  a) isolating an immunocompetent cell taken from a donor animal immunized beforehand,
  b) fusing said immunocompetent cell with a tumour cell line capable of continuous cell division, and
  c) isolating the resulting fusion product.

**7.** The method according to claim 6, wherein said immunocompetent cell is a spleen cell.

**8.** The method according to claim 6, wherein said tumour cell line is a myeloma cell line which does not itself produce monoclonal antibodies.

**9.** The method according to claim 8 wherein the myeloma cell line is SP2-O-Ag14 or X63-Ag8.653.

**10.** A method of producing a monoclonal antibody according to claim 3, which comprises _in vitro_ or _in vivo_ mass cultivation of a hybridoma which produces a monoclonal antibody which reacts with at least one pathogenic strain of the genus Phytophthora but which exhibits substantially no reaction with strains of the genus Pythium and is capable of detecting Phytophthora in diseased tissue in a double-antibody immunoassay.

**11.** The method according to claim 18, wherein a hybridoma according to one of claims 2 to 5 is mass cultured.

**12.** A method of detecting the presence of Phytophthora antigen in a sample containing said antigen, which method comprises the measures of:
  a) forming a binary complex between the antigen in the sample and a first monoclonal or polyclonal antibody capable of reacting with said antigen;
  b) forming a tertiary complex by contacting the binary complex with a second monoclonal or polyclonal antibody capable of reacting with said antigen; and
  c) detecting the presence of a tertiary complex on the basis of a detectable signal produced by an analytically detectable reagent, which reagent optionally may be conjugated to a third antibody;
  where at least one of the antibodies is a monoclonal antibody which reacts with at least one pathogenic strain of the genus Phytophthora but which exhibits substantially no reaction with strains of the genus Pythium and is capable of detecting Phytophthora in diseased tissue in a double-antibody immunoassay.

**13.** The method according to claim 12, wherein the detectable reagent is conjugated to the second antibody.

**14.** The method according to claim 12, wherein the detectable reagent is conjugated to a third, anti-immunoglobulin antibody which is contacted with the tertiary complex.

**15.** The method according to claim 12, wherein said reagent is an enzyme, a radioisotope, a fluorophore or biotin.

**16.** The method according to claim 14, wherein said reagent is an enzyme, a radioisotope, a fluorophore or biotin.

**17.** The method according to claim 12, wherein one of the antibodies is immobilized on a solid support.

**18.** A test kit for the immunological diagnosis of Phytophthora infections in plants, comprising a carrier which is compartmentalized so as to receive, in close mutual confinement therein:
  a) a solid support having affixed thereto a first monoclonal or polyclonal antibody capable of reacting with Phytophthora; and
  b) a detection system for a binary complex, said system comprising a second monoclonal or polyclonal antibody as well as optionally a third antibody;

EP 0 302 011 B1

where at least one of the antibodies is a monoclonal antibody which reacts with at least one pathogenic strain of the genus Phytophthora but which exhibits substantially no reaction with strains of the genus Pythium and is capable of detecting Phytophthora in diseased tissue in a double-antibody immunoassay.

19. The test kit according to claim 18, wherein the second antibody is conjugated to an analytically detectable reagent selected from the group consisting of an enzyme, a radioisotope, a fluorophore or biotin.

20. The test kit according to claim 18, wherein the kit comprises a third, anti-immunoglobulin antibody conjugated to an analytically detectable reagent selected from the group consisting of an enzyme, a radioisotope, a fluorophore or biotin.

**Claims for the following Contracting State : ES**

1. A method of producing a hybridoma which produces a monoclonal antibody which reacts with at least one pathogenic strain of the genus Phytophthora but which exhibits substantially no reaction with strains of the genus Pythium and is capable of detecting Phytophthora in diseased tissue in a double-antibody immunoassay, which comprises
   a) isolating an immunocompetent cell taken from a donor animal immunized beforehand,
   b) fusing said immunocompetent cell with a tumour cell line capable of continuous cell division, and
   c) isolating the resulting fusion product.

2. The method according to claim 1, wherein said immunocompetent cell is a spleen cell.

3. The method according to claim 1, wherein said tumour cell line is a myeloma cell line which does not itself produce monoclonal antibodies.

4. The method according to claim 3, wherein the myeloma cell line is SP2-O-Ag14 or X63-Ag8.653.

5. The method according to claim 1, wherein said hybridoma produces a monoclonal antibody which belongs to the IgG2b subclass.

6. A method of producing a monoclonal antibody which reacts with at least one pathogenic strain of the genus Phytophthora but which exhibits substantially no reaction with strains of the genus Pythium and is capable of detecting Phytophthora in diseased tissue in a double-antibody immunoassay, which comprises in vitro or in vivo mass cultivation of a hybridoma according to one of claims 1 to 5.

7. The method according to claim 6 wherein said monoclonal antibody belongs to the IgG2b subclass.

8. A method of detecting the presence of Phytophthora antigen in a sample containing said antigen, which method comprises the measures of:
   a) forming a binary complex between the antigen in the sample and a first monoclonal or polyclonal antibody capable of reacting with said antigen;
   b) forming a tertiary complex by contacting the binary complex with a second monoclonal or polyclonal antibody capable of reacting with said antigen; and
   c) detecting the presence of a tertiary complex on the basis of a detectable signal produced by an analytically detectable reagent, which reagent optionally may be conjugated to a third antibody;
   where at least one of the antibodies is a monoclonal antibody which reacts with at least one pathogenic strain of the genus Phytophthora but which exhibits substantially no reaction with strains of the genus Pythium and is capable of detecting Phytophthora in diseased tissue in a double-antibody immunoassay.

9. The method according to claim 8, wherein the detectable reagent is conjugated to the second antibody.

10. The method according to claim 8, wherein the detectable reagent is conjugated to a third, anti-immunoglobulin antibody which is contacted with the tertiary complex.

23

**11.** The method according to claim 8, wherein said reagent is an enzyme, a radioisotope, a fluorophore or biotin.

**12.** The method according to claim 10, wherein said reagent is an enzyme, a radioisotope, a fluorophore or biotin.

**13.** The method according to claim 10, wherein one of the antibodies is immobilized on a solid support.

**14.** A means for the immunological diagnosis of Phytophthora infections in plants, in the form of a ready-to-use test kit comprising a carrier which is compartmentalized so as to receive, in close mutual confinement therein:

a) a solid support having affixed thereto a first monoclonal or polyclonal antibody capable of reacting with Phytophthora; and

b) a detection system for a binary complex, said system comprising a second monoclonal or polyclonal antibody as well as optionally a third antibody;

where at least one of the antibodies is a monoclonal antibody which reacts with at least one pathogenic strain of the genus Phytophthora but which exhibits substantially no reaction with strains of the genus Pythium and is capable of detecting Phytophthora in diseased tissue in a double-antibody immunoassay.

**15.** The means according to claim 14, wherein the second antibody is conjugated to an analytically detectable reagent selected from the group consisting of an enzyme, a radioisotope, a fluorophore or biotin.

**16.** The means according to claim 14, wherein the kit comprises a third, anti-immunoglobulin antibody conjugated to an analytically detectable reagent selected from the group consisting of an enzyme, a radioisotope, a fluorophore or biotin.

**Revendications**

**Revendications pour les Etats contractants suivants : AT, BE, CH, LI, DE, FR, GB, GR, IT, LU, NL, SE**

**1.** Hybridome produisant un anticorps monoclonal qui réagit avec au moins une souche pathogène du genre Phytophtora, mais qui ne présente essentiellement pas de réaction avec les souches du genre Pythium et est en situation de mettre en évidence Phytophtora dans un tissu malade dans un immunoessai comprenant deux anticorps.

**2.** Hybridome selon la revendication 1, caractérisé en ce que l'anticorps monoclonal mentionné appartient à la sous-classe IgG2b.

**3.** Anticorps monoclonal qui réagit avec au moins une souche pathogène du genre Phytophtora mais qui ne présente essentiellement pas de réaction avec les souches du genre Pythium et est en situation de mettre en évidence Phytophtora dans le tissu malade dans un immunoessai comprenant deux anticorps.

**4.** Anticorps selon la revendication 3, caractérisé en ce qu'il appartient à la sous-classe IgG2b.

**5.** Anticorps monoclonal préparé à partir d'un hybridome selon la revendication 1 ou 2.

**6.** Procédé de préparation d'un hybridome selon la revendication 1, caractérisé en ce que :

a) on isole une cellule immunocompétente à partir d'un animal donneur préalablement immunisé,

b) on la fusionne avec une lignée cellulaire tumorale capable de division cellulaire continue, et

c) on isole le produit de fusion apparu.

**7.** Procédé selon la revendication 6, caractérisé en ce que, pour la cellule immunocompétente mentionnée, il s'agit d'une cellule de rate.

**8.** Procédé selon la revendication 6, caractérisé en ce que, en ce qui concerne la lignée cellulaire tumorale mentionnée, il s'agit d'une lignée cellulaire de myélome qui ne produit pas le même anticorps monoclonal.

9. Procédé selon la revendication 8, caractérisé en ce qu'il s'agit de la lignée cellulaire de myélome SP2-O-Ag14 ou X63-Ag8.653.

10. Procédé de préparation d'un anticorps monoclonal selon la revendication 3, caractérisé en ce qu'on effectue une culture en masse in vitro ou in vivo d'un hybridome qui produit un anticorps monoclonal réagissant avec au moins une souche pathogène du genre Phytophtora, mais ne présentant essentiellement pas de réaction avec les souches du genre Pythium et qui est en situation de mettre en évidence Phytophtora dans le tissu malade dans un immunoessai comprenant deux anticorps.

11. Procédé selon la revendication 10, caractérisé en ce qu'on cultive en masse un hybridome selon l'une des revendications de 2 à 5,

12. Procédé de mise en évidence de la présence d'un antigène Phytophtora dans un échantillon contenant un antigène mentionné, qui se caractérise par les mesures opératoires suivantes:
    a) formation d'un complexe binaire entre l'oxygène dans l'échantillon et un premier anticorps monoclonal ou polyclonal, qui est en situation de réagir avec l'antigène mentionné;
    b) formation d'un complexe tertiaire en amenant en contact le complexe binaire avec un second anticorps monoclonal ou polyclonal qui est en situation de réagir avec l'antigène mentionné et
    c) mise en évidence de la présence d'un complexe tertiaire sur la base de son signal perceptible, qui est produit par un réactif pouvant être mis en évidence par analyse; qui peut le cas échéant se présenter conjugué à un troisième anticorps; où
    au moins un des anticorps est un anticorps monoclonal qui réagit avec au moins une souche pathogène du genre Phytophtora mais qui ne présente esentiellement pas de réaction avec la souche du genre Pythium et est en situation de mettre en évidence Phytophtora dans le tissu malade dans un immunoessai comprenant deux anticorps.

13. Procédé selon la revendication 12, caractérisé en ce que le réactif pouvant être mis en évidence se présente conjugué au second anticorps.

14. Procédé selon la revendication 12, caractérisé en ce que le réactif pouvant être mis en évidence se présente conjugué à un troisième anticorps anti-immunoglobuline qui est mis en contact avec le complexe tertiaire.

15. Procédé selon la revendication 12, caractérisé en ce que, en ce qui concerne le réactif mentionné, il s'agit d'une enzyme, d'un radioisotope, d'un fluorophore ou de la biotine.

16. Procédé selon la revendication 14, caractérisé en ce que, en ce qui concerne le réactif mentionné, il s'agit d'une enzyme, d'un radioisotope, d'un fluorophore ou de la biotine.

17. Procédé selon la revendication 12, caractérisé en ce que l'un des anticorps est présent immobilisé sur un support solide.

18. Nécessaire de test pour le diagnostic immunologique des injections à Phytophtora chez des plantes, caractérisé par un support qui est compartimenté si bien qu'on y trouve disposés dans un rapport spatial étroit:
    a) un support solide qui contient, lié à un premier anticorps monoclonal ou polyclonal qui est en situation de réagir avec Phytophtora; et
    b) un système de mise en évidence pour complexe binaire, qui contient un second anticorps monoclonal ou polyclonal ainsi que le cas échéant un troisième anticorps;
    où au moins l'un des anticorps est un anticorps monoclonal qui réagit avec au moins une souche pathogène du genre Phytophtora, mais qui ne présente essentiellement pas de réaction avec les souches du genre Pythium et est en situation de mettre en évidence Phytophtora dans le tissu malade dans un immunoessai comprenant deux anticorps.

19. Nécessaire de test selon la revendication 18, caractérisé en ce que le second anticorps est conjugué à un réactif pouvant être mis en évidence par analyse, choisi dans le groupe constitué par une enzyme, un radioisotope, un fluorophore ou la biotine.

**20.** Nécessaire de test selon la revendication 18, caractérisé en ce que le nécessaire contient un troisième anticorps anti-immunoglobuline qui est conjugué à un réactif pouvant être mis en évidence par analyse et choisi dans le groupe constitué par une enzyme, un radioisotope, un fluorophore ou la biotine.

**Revendications pour l'Etat contractant suivant : ES**

**1.** Procédé de préparation d'un hybridome qui produit un anticorps monoclonal réagissant avec au moins une souche pathogène du genre Phytophtora mais ne présentant essentiellement pas de réaction avec les souches du genre Pythium et qui est en situation de mettre en évidence Phytophtora dans le tissu malade dans un immunoessai comprenant deux anticorps, caractérisé en ce que
   a) on isole une cellule immunocompétente à partir d'un animal donneur préalablement immunisé;
   b) on la fusionne avec une lignée cellulaire tumorale apte à la division cellulaire continue ; et
   c) on isole le produit de fusion apparu.

**2.** Procédé selon la revendication 1, caractérisé en ce que, en ce qui concerne la cellule immunocompétente mentionnée, il s'agit d'une cellule de rate.

**3.** Procédé selon la revendication 1, caractérisé en ce que, pour ce qui est de la lignée cellulaire tumorale mentionnée, il s'agit d'une lignée cellulaire de myélome qui ne produit pas elle-même d'anticorps monoclonaux.

**4.** Procédé selon la revendication 3, caractérisé en ce qu'il s'agit de la lignée cellulaire de myélome KSP2-O-Ag14 ou X63-Ag8.653.

**5.** Procédé selon la revendication 1, caractérisé en ce que l'hybridome mentionné produit un anticorps monoclonal qui appartient à la sous-classe IgG2b.

**6.** Procédé de préparation d'un anticorps monoclonal qui réagit avec au moins une souche pathogène du genre Phytophtora, mais qui ne présente essentiellement pas de réaction avec les souches du genre Pythium et est en situation de mettre en évidence Phytophtora dans le tissu malade dans un immunoessai comprenant deux anticorps, caractérisé en ce qu'on cultive en masse un hybridome selon l'une des revendications 1 à 5, in vitro ou in vivo.

**7.** Procédé selon la revendication 6, caractérisé en ce que l'anticorps monoclonal mentionné appartient à la sous-classe IgG2b.

**8.** Procédé de mise en évidence de la présence d'antigènes de Phytophtora dans un échantillon contenant l'antigène mentionné, caractérisé par les mesures opératoires suivantes:
   a) formation d'un complexe binaire entre l'oxygène dans l'échantillon et un premier anticorps monoclonal ou polyclonal, qui est en situation de réagir avec l'antigène mentionné;
   b) formation d'un complexe tertiaire en amenant en contact le complexe binaire avec un second anticorps monoclonal ou polyclonal qui est en situation de réagir avec l'antigène mentionné et
   c) mise en évidence de la présence d'un complexe tertiaire sur la base de son signal perceptible, qui est produit par un réactif pouvant être mis en évidence par analyse; qui peut le cas échéant se présenter conjugué à un troisième anticorps; où
   au moins un des anticorps est un anticorps monoclonal qui réagit avec au moins une souche pathogène du genre Phytophtora mais qui ne présente esentiellement pas de réaction avec la souche du genre Pythium et est en situation de mettre en évidence Phytophtora dans le tissu malade dans un immunoessai comprenant deux anticorps.

**9.** Procédé selon la revendication 8, caractérisé en ce que le réactif pouvant être mis en évidence se présente conjugué au second anticorps.

**10.** Procédé selon la revendication 8, caractérisé en ce que le réactif pouvant être mis en évidence se présente conjugué à un troisième anticorps anti-immunoglobuline qui est mis en contact avec le complexe tertiaire.

**11.** Procédé selon la revendication 8, caractérisé en ce que, en ce qui concerne le réactif mentionné, il s'agit d'une enzyme, d'un radioisotope, d'un fluorophore ou de la biotine.

**12.** Procédé selon la revendication 10, caractérisé en ce que, en ce qui concerne le réactif mentionné, il s'agit d'une enzyme, d'un radioisotope, d'un fluorophore ou de la biotine.

**13.** Procédé selon la revendication 10, caractérisé en ce que l'un des anticorps soit présent immobilisé sur un support solide.

**14.** Agent de diagnostic immunologique des infections à Phytophtora dans les plantes sous forme d'un nécessaire de test prêt à l'emploi, qui se caractérise par un support qui est compartimenté de manière qu'on y trouve disposés dans un rapport spatial étroit:
a) un support solide qui contient, lié à un premier anticorps monoclonal ou polyclonal qui est en situation de réagir avec Phytophtora; et
b) un système de mise en évidence pour complexe binaire, qui contient un second anticorps monoclonal ou polyclonal ainsi que le cas échéant un troisième anticorps;
où au moins l'un des anticorps est un anticorps monoclonal qui réagit avec au moins une souche pathogène du genre Phytophtora, mais qui ne présente essentiellement pas de réaction avec les souches du genre Pythium et est en situation de mettre en évidence Phytophtora dans le tissu malade dans un immunoessai comprenant deux anticorps.

**15.** Agent selon la revendication 14, caractérisé en ce que le second anticorps est conjugué à un réactif pouvant être mis en évidence par analyse, choisi dans le groupe constitué par une enzyme, un radioisotope, un fluorophore ou la biotine.

**16.** Agent selon la revendication 14, caractérisé en ce que le nécessaire contient un troisième anticorps antiimmunoglobuline qui est conjugué à un réactif pouvant être mis en évidence par analyse et choisi dans le groupe constitué par une enzyme, un radioisotope, un fluorophore ou la biotine.